# EUROPEAN PATENT APPLICATION

(11) **EP 1 319 423 A2**
(43) Date of publication of application: **18.06.2003**
(21) Application number: 02027643.2
(22) Date of filing: 11.12.2002
(51) Int. Cl.: A61N 7/00

(54) **Apparatus and method for initiating chemical reactions and for the targeted delivery of drugs or other agents**

(30) Priority: 11.12.2001 US 339285 P
(71) Applicant: Dornier Medtech System GmbH, 82234 Wessling (DE)
(72) Inventor: Ueberle, Friedrich, 82205 Gilching (DE)
(74) Representative: Grünecker, Kinkeldey, Stockmair & Schwanhäusser Anwaltssozietät

(57) **Abstract**

The present invention is directed to an apparatus and a method for targeted activation or deactivation of chemical reactions, comprising initiation means for initiating cavitation inside at least one medium, wherein the initiation means are adapted to generate photons by means of controlled cavitation for the activation or deactivation of chemical reactions within the at least one medium.

## Description

The current invention relates to an apparatus and method for targeted activation or deactivation of chemical reactions in a medium.

In molecules, chemical bonds may be modeled as elastic springs, which connect two atoms. The springs have a specific energy content. To achieve a chemical reaction, it is necessary to break up these bonds. Depending on the reaction pattem, the necessary energy for this process may be applied in the form of thermal energy or physical energy (i.e. shaking). Less traditional approaches have utilized photoenergy, i.e., laser light. For example, continuous light of a certain wavelength is used to achieve a resonance phenomenon. The bonded atoms oscillate at a typical frequency determined by the atomic mass and the "spring constant" of the bond. At resonance, this leads to excessive oscillations, which finally result in the breaking of the bonds.

Although this model functions well in theory, there are practical limitations in real-world systems. In molecules, the atoms are also influenced by their environment and do not oscillate independently. It is known that the actual breaking of bonds happens in a very short time, usually some hundred femtoseconds. Very short time laser pulses have been demonstrated to initiate the breaking process (Assion et al, 1998, Science 282: 919-922).

However, the use of laser energy has a very limited usage for breaking chemical bonds or initiating chemical reaction in a clinical setting. For example, laser energy has only a limited ability to penetrate tissue. In particular, the wave shapes of laser pulses may be changed significantly even at short distances while passing through even through relatively short distance of tissue layers. Thus, laser energy would have limited practicality in targeting a reaction even within several centimeters of the tissue surface.

In contrast, it has been demonstrated that mechanical energies, such as those generated by sound, are able to penetrate almost every part of the human body. Examples, include, the ultrasound imaging of organs and the extracorporeally-induced disintegration of kidney or gall stones by pressure pulses. Sound or acoustical energy is unique in that cavitation, which is the occurrence of bubbles in fluids, may be generated by rarefaction phases of sound signals. Pre-existing cavitation seeds at the target for example, are agitated by the sound waves. During rarefaction phases of the sound, the seeds or bubbles begin to expand expansion. After reaching a quasi-stable size around the maximum radius of the bubbles, they collapse. When collapsing, the stored energy is released as a burst of energy comprising acoustic energy in the form of a shock wave. At higher thresholds, energy is released in the form of a shock wave and electromagnetic energy in the form of a light flash.

For inducing cavitation inside a host conventional ultrasonic devices or shock wave devices have been used which have a rarefaction phase or a tensile wave component with about -10 MPa in water. However, conventional shock wave devices are typically designed in such a way that the tensile wave component is as small in order to avoid the induction of cavitation.

It is known from the prior art that chemical reactions may be initiated or deactivated by electromagnetic radiation and in particular by electromagnetic radiation in the infrared, visible or ultraviolet range. Although chemical substances may be delivered nearly to any place in a host, the delivery of light through the host is limited by the absorption and the penetration depth of the host target. Therefore, conventional treatment methods using light for activating or deactivating chemical substances are limited to a region very close to the surface of a host or body. Alternatively it is possible to transmit the necessary light via a catheter or an optical fiber to a target region inside a host or a body, however, the therapy range is still limited by the absorption or penetration depth in accordance of the wavelength.

### Targeted Drug Delivery

One of the holy grails of pharmacological therapeutics is the targeted, precise delivery of a drug or other biological agent to a specific diseased site within an organism. Virtually all drugs are capable of eliciting side and/or toxic effects, which often limit their usefulness. These effects are typically due to the drug or agent's effects upon organ systems or tissue other than the one being targeted by that particular drug. For example, a drug that targets a particular receptor in one part of the body will necessarily end up targeting those particular receptors throughout the body. Delivery of a drug or other agent to the precise desired target would virtually eliminate self-limiting side or toxic effects of drugs. Furthermore, if a drug could be precisely targeted to a desired locale within the organism, much lower concentrations would normally have to be administered since the clinician would not have to take into consideration such pharmacological variables as distribution and elimination of the drug.

Methods to precisely deliver drugs have been attempted, typically with limited success. For example, although the use of monoclonal antibody technology once held great promise as a means to specifically target drugs or other agents in the body, it has not met with much practical success. Encapsulating drugs or other agents with such materials as polymers, liposomes and the like allows for the delivery of agents that typically can be very toxic to the individual (i.e. anti-cancer agents) or for materials that would easily break down with normal routes of administration (i.e. proteins or peptides).

U.S. Patent No. 6,444,217 to Kwok et al discloses an implantable drug delivery device that posses a surface layer adapted to retain and controllably release drug molecules wherein the device is comprised of a polymer encapsulated drug or pharmaceutical agent.

U.S. Patent No. 6,465,006 to Zhang et al discloses a dermal drug delivery system that uses thermal energy to facilitate the delivery of drugs within the organism.

U.S. Patent Nos. 6,443,898, 6,461,586 and 6,416,740 describe the use of gas-filled microspheres that include a therapeutic agent for drug delivery and magnetic resonance imaging within a patient. These patents disclose the mechanical disruption of microspheres within the patient by a variety of different energy means including acoustic waves via the release of shock waves from the collapse of cavitational bubbles at low cavitational thresholds. U.S. Patent No. 6,416,740 discloses a targeted therapeutic delivery system gas filled microsphere to which is added a therapeutic compound. U.S. Patent No. 6,443,898 claims a method for the controlled delivery of a therapeutic compound to a specific region of a patient where the compound is contained within a temperature activated gas-filled microsphere. The patent discloses that the microspheres are mechanically ruptured by applications of various types of energy including ultrasound, microwaves, magnetic induction oscillating energy and light energy. U.S. Patent No. 6,461,586 claims a method for the control delivery of a therapeutic compound using magnetic resonance focused therapeutic ultrasound to mechanically rupture vesicles containing a therapeutic compound.

U.S. Patent Nos. 5,498,421, 5,635,207, 5,639,473, 5,650,156, 5,665,382 and 5,665,383 to Grinstaff et al disclose compositions comprising a therapeutic agent associated with a polymeric shell for in vivo drug delivery. The therapeutic agents disclosed in these patents include, drugs, pharmaceutical agents, immunostimulatory substances, nucleic acids and nutriceuticals.

U.S. Patent Nos. 5,531,980, 5,567,414, 5,543,553, and 5,658,551 to Schneider et al disclose the use of gas or air filled microbubble suspensions comprising for example liposomes for use as imaging contrast agents capable of being injected into an organism.

U.S. Patent No.5,795,581 to Segalman et al discloses the use of dendrimers host molecules, which form a matrix around a desired therapeutic agent for in vivo delivery. The therapeutic agent is release by electromagnetic radiation.

WO0076406 teaches an ultrasonic method and kit to accelerate healing of bone and tissue injuries. It teaches that the cavitation effects caused by the disclosed method and claims devices can increase the permeability of a cellular wall membrane. The device can be inserted intracorporally or implanted into the patient.

WO0069942 teaches the controlled release of drugs such as anti-cancer agents encapsulated within micelles using pulsed ultrasound.

WO99/42176 and WO99/42039 disclose general methods for as well as an endoluminal implant that can comprise an ultrasonic transducer that can be used to mechanically rupture delivery vehicles for localized drug delivery.

WO00/02588 teaches a method for the delivery of therapeutic compounds, encapsulated within a protein microbubble wherein targeted ultrasound is used to promote the release of the agent.

Methods, which utilize acoustic energy to initiate cavitation at low cavitation thresholds, suffer from the constraint that the absorption or penetration depth of the applied acoustic energy is limited.

Therefore, it is an object of the present invention to provide a system and method which allows the activation or deactivation of chemical reactions via electromagnetic radiation within the IR, visible or UV range, wherein this initiation or deactivation of chemical reactions inside a host or body, in which the chemical reactions should be initiated or deactivated, is not limited to a region of the host or body, which is accessible for a source of the electromagnetic radiation in respect to the absorption or penetration depth of the host or body.

The inventors of the present invention have unexpectedly found that the generation of cavitation by an acoustic device at a peak tensile pressure of -5 MPa to -15 MPa allows the initiation or deactivation of chemical reactions via electromagnetic radiation within the infrared, visible or ultraviolet range, and that these chemical reactions can be initiated or deactivated using this technique without being limited by the absorption or penetration depth of the electromagnetic wave inside the host. For inducing cavitation inside a host conventional ultrasonic devices or shock wave devices have been used which have a rarefaction phase or a tensile wave component with about -10 MPa in water. However, conventional shock wave devices are designed in such a way that the tensile wave component is as small as possible in order to avoid the cavitation. In contrast, the inventors of the present invention have discovered that when a maximal cavitation effect is produced, sonoluminescence, occurs in which the tensile wave component is produced first followed by the high pressure component. This is the opposite effect of a conventional shock wave, where the high pressure part comes first followed by the tensile wave component. The inventors have found that the optimal effects occur at peak tensile pressure of these acoustic waves in the range of -5 MPa to -15 MPa. They have discovered that the invention disclosed herein combines the processes of electromagnetic excitation of molecules for targeted initiation or deactivation of chemical reactions together with a shock wave induced permeability of a cell membrane.

Thus, the present invention is directed to methods and apparatus for the targeted initiation or deactivation of chemical reactions by an acoustic energy source in a host. Methods and apparatus for the targeted delivery of drugs, diagnostic agents and other compounds using an acoustic energy source are also provided.

The invention provides an apparatus and a method for targeted activation or deactivation of chemical reactions, comprising initiation means for initiating cavitation inside at least one medium, wherein the initiation means are adapted to generate photons by means of controlled cavitation for the activation or deactivation of chemical reactions within the at least one medium.

The cavitation process produces cavitation bubbles that emit ultra short light pulses upon collapse within the infrared, visible and ultraviolet range which in turn activate or deactivate chemical reactions that are sensitive to ultra short light pulses within the infrared, visible or ultraviolet ranges. In one aspect of the invention, the chemical reaction comprises the three-dimensional folding or assembly of a drug or biologic agent. In another aspect of the invention, the drug or biologic agent combines with one or more ions or free radicals generated during the collapse of the cavitation bubbles.

One embodiment provides an apparatus for targeted activation or deactivation of chemical reactions, comprising initiation means for initiating cavitation inside at least one medium, wherein the initiation means are adapted to generate photons by means of controlled cavitation for the activation or deactivation of chemical reactions within the at least one medium.

A further embodiment provides an implantable drug delivery device comprising a miniaturized electronic circuit chip, a sensor, a drug or agent storage means and a means for the generation of the acoustic waves at a specific peak tensile pressure to induce cavitation.

Another embodiment provides an implantable bio-sensing device comprising a miniaturized electronic circuit chip, a sensor, a means for the generation of the acoustic waves at a specific peak tensile pressure to induce cavitation inside a channel, wherein the channel guides a fluid containing a substance or cells to be analyzed.

Certain embodiments of the invention provide that the chemical reaction is the activation of a prodrug, active drug or biological agent which can then enter a cell, tissue or act upon a specific site with in the host.

The invention further provides that a compound or substance that enhances the cavitation process can be included as part of the chemical reaction or be delivered with the drug or biologic agent. In one aspect, the prodrug, drug or biological agent itself enhances the cavitation process. In other aspects, the compound or substance that enhances the cavitation process includes gas filled microspheres, such as a liposome, ultrasonic contrast agents, any inorganic gas filled molecule that does not reflect, any material with a low acoustic impedance such as a plasticbead, a lipid or oil droplet, and any material with a high acoustic impedance including an inert material such as small gold pellets. In other embodiments, the methods of the invention are combined with an imaging method such as magnetic resonance imaging and the like.

In one embodiment the acoustic waves of the invention are applied to the host from an external energy source. In other embodiments the acoustic waves are produced by an optical fiber inserted into a blood vessel or orifice of the host. The acoustic waves may be optionally guided inside the host by a sound guiding means.

The invention may be practiced across a range of conditions by varying the gas content of the cavitation bubbles, which in turn influence the spectral content of the electromagnetic energy induced by the cavitation. The spectral content of the cavitation collapse light can be changed by supplying an appropriate gas inside the compound or a substance that enhances the cavitation process. Alternatively, the spectral content of the cavitation collapse light can be changed by adding an appropriate gas to the normal breathing gas of the host.

Drugs or biologic agents that can be delivered to the host include prodrugs, targeting ligands, diagnostic agents, pharmaceutical agents, drugs, synthetic organic molecules, proteins, peptides, vitamins, steroids, steroid analogs and genetic material. Targeting ligands which are used to direct the desired drug or biological agent to a specified organ, tissue or cell can include proteins, antibodies, antibody fragments, hormones, hormone analogues, glycoproteins, lectins, peptides, polypeptides, amino acids, sugars, monosaccharides, polysaccharides, carbohydrates, vitamins, steroids, steroid analogs, cofactors, and genetic material such as DNA, RNA, mRNA, cDNA, nucleosides, nucleotides, nudeotide acid constructs and polynucleotides.

The drug or biological agent can be optionally encapsulated within a substance or compound to facilitate targeted delivery, prevent unwanted toxicity or allow the use of decreased dosages to be used. Such encapsulating materials include but are not limited to, gas-filled microspheres, clathrates, polymer shells, and liposomes.

In various embodiments of the invention, the host's blood is directed outside the host to a fluid chamber at the focus of the acoustic source. The inactivated drug or biological agent is then mixed with the host's blood in the fluid chamber and the subsequent mixture is then guided through the focus of the acoustic source, where it receives the acoustic pulses in order to activate the drug or biological agent. The blood is then redirected to the host. A compound or substance that enhances the cavitation process can be optionally added to the fluid chamber.

An apparatus for initiating or deactivating chemical reactions at a target inside a host is also provided that includes a means for generating acoustic waves of a sufficient amplitude to induce cavitation, wherein the chemical reactions are sensitive to ultra short light pulses within the infrared, visible or ultraviolet range, and wherein cavitation bubbles are produced that emit ultra short light pulses upon collapse within the infrared, visible and ultraviolet range. In one embodiment, the apparatus or device can optionally direct the host's blood outside the host to be mixed with drug or biological agent in a fluid chamber at the focus of the acoustic source.

The present invention also provides for an implantable drug delivery device that includes a miniaturized electronic circuit chip, a sensor, a drug or agent storage means as well as a means for the generation of the acoustic waves inside a channel, wherein the channel guides a fluid containing the drug or agent into the host. This device can optionally include a second acoustic wave generator capable of generating a cavitational zone to activate or deactivate the drug or agent.

Similarly, an implantable bio-sensing device is also provided that includes a miniaturized electronic circuit chip, a sensor as well as a means for the generation of the acoustic waves inside a channel, wherein the channel guides a fluid containing a substance or cells to be analyzed. This device can be optionally equipped with a second acoustic wave generator capable of forming a cavitational zone close to the sensor. A channel then guides a fluid or cells through the cavitational zone close to the sensor in order to detect properties of the fluid or cells, which are agitated by the cavitational effects close to the sensor area.

The embodiments described above have the advantage that due to an acoustic wave induced sonoluminescence the light can be generated nearly everywhere inside a medium without the necessity of penetrating the medium with a catheter or optical fiber. Furthermore, by focusing the acoustic waves to a certain region of the medium the activation or deactivation of chemical reactions is limited to the region of the focus of the acoustic waves. Additionally, the invention advantageously provides the possibility to combine the initiation of chemical reactions by electromagnetic radiation and acoustic waves in one apparatus.

These and other aspects and advantages will be apparent from the following detailed description of the invention.

The present invention is directed to methods for the targeted initiation or deactivation of chemical reactions in a host, and to apparatus for performing such methods. Methods for delivery of drugs, diagnostic agents and other compounds using an acoustic energy source are also provided.

The preferred embodiment is an apparatus for targeted activation or deactivation of chemical reactions, which comprises initiation means for initiating cavitation inside a medium. The initiation means are adapted to generate photons by means of controlled cavitation for the activation or deactivation of chemical reactions within the medium.

This basic aspect of the invention of generating sonoluminescence with shock waves inside a body or host and using the thus generated ultra short light pulses for initiating or deactivating chemical reactions is combined with a variety of different chemical substances for treatment together with a variety of delivery methods for these chemicals substances.

The device for generating acoustic waves, which is a conventional lithotripter in an alternative embodiment, generates acoustic waves with such an amplitude that cavitation is induced inside a medium wherein due to the cavitation collapsing bubbles are generated which emit electromagnetic radiation within the IR, visible and UV range and wherein the thus generated light is used for initiating or deactivating chemical reactions inside the medium.

The acoustic waves are preferably generated with at least one device for generating the acoustic waves using an electro hydraulic, piezoelectric or electromagnetic effect. Preferably, the device for generating the acoustic waves is a non-invasive device.

Alternatively, the device for generating cavitation inside a medium is a miniaturized device, which is placed inside the host or body. One example for the miniaturization of such a device is a specially prepared optical fiber, which is used for generating cavitation inside the host or body. For this shock waves are guided through the optical fiber or shock waves are induced at the end of the optical fiber by laser pulses. One example for this is a fiber used for dissolving blood clots within the heart region. Such an optical fiber is usable in various ways to generate acoustic waves by inserting it into a blood vessel.

A further alternative to the above described embodiment is a sound guiding means for guiding acoustic waves inside a medium

The acoustic waves are focused on an area within the medium, where chemical reactions shall be activated or deactivated by the cavitation induced sonoluminescence. Therefore, the sonoluminescence is mainly limited to a region around the focus of the acoustic waves. Thus, the volume for the targeted activation or deactivation of the chemical reactions is controlled by the size of the focus of the acoustic waves.

Alternatively, the acoustic waves are not focused in order to activate or deactivate chemical reactions within a maximum possible region of the medium.

The medium, wherein the cavitation is induced, consists of fluids or matters containing fluid substances. Alternatively, the medium is a chemical reactor containing fluids or fluid substances, a plant, a body or a part of a body.

### I. Definitions

"Biological" or "bioactive agent" refers to any substance that can elicit an effect on a biological system in the host. Such agents include but are not limited to, proteins, peptides, polypeptides, antibodies, antigenic material, receptors, receptor subunits, neurotransmitters, neuromodulators, steroids, DNA, RNA, sense or antisense strands, nucleotides, nucleosides, vaccines, viruses or particles of viruses, bacteria or particles of bacteria, lipids, carbohydrates, sugars, ions, metals, prodrugs, targeting ligands, diagnostic agents, pharmaceutical agents, drugs, synthetic organic molecules, vitamins, and the like.

"Carrier" refers to a pharmaceutically acceptable vehicle, which is a nonpolar, hydrophobic solvent, and which may serve as a reconstituting medium. The carrier may be aqueous-based or organic-based. Carriers include, but are not limited to, lipids, proteins, polysaccharides, sugars, polymers, copolymers, and acrylates.

"Lipid" refers to a naturally-occurring, synthetic or semi-synthetic (i.e., modified natural) compound, which is generally amphipathic. The lipids typically comprise a hydrophilic component and a hydrophobic component. Exemplary lipids include, for example, fatty acids, neutral fats, phosphatides, oils, glycolipids, surface-active agents (surfactants), aliphatic alcohols, waxes, terpenes and steroids. The phrase semi-synthetic (or modified natural) denotes a natural compound that has been chemically modified in some fashion.

"Polymer" or "polymeric" refers to molecules formed from the chemical union of two or more repeating units or monomers.

"Emulsion" refers to a mixture of two or more generally immiscible liquids, and is generally in the form of a colloid. The mixture may be of lipids, for example, which may be homogeneously or heterogeneously dispersed throughout the emulsion. Alternatively, the lipids may be aggregated in the form of, for example, clusters or layers, including monolayers or bilayers.

"Stabilizing material" or "stabilizing compound" refers to any material which is capable of improving the stability of compositions containing the gases, gaseous precursors, drugs, prodrugs, targeting ligands and/or other biological agents described herein, including, for example, mixtures, suspensions, emulsions, dispersions, vesicles, or the like. Encompassed in the definition of "stabilizing material" are certain of the disclosed drugs or biological agents. The improved stability involves, for example, the maintenance of a relatively balanced condition, and may be exemplified, for example, by increased resistance of the composition against destruction, decomposition, degradation, and the like. In the case of preferred embodiments involving vesicles filled with gases, gaseous precursors, liquids, drugs, prodrugs and/or bioactive agents, the stabilizing compounds may serve to either form the vesicles or stabilize the vesicles, in either way serving to minimize or substantially (including completely) prevent the escape of gases, gaseous precursors, drug, prodrugs and/or bioactive agents from the vesicles until said release is desired. Stabilizing materials include, but are not limited to, lipids, proteins, polymers, carbohydrates and surfactants. The resulting mixture, suspension, emulsion or the like may comprise walls (i.e., films, membranes and the like) around the steroid prodrug, bioactive agent, gases and/or gaseous precursors, or may be substantially devoid of walls or membranes, if desired. The stabilizing material can also form droplets. The stabilizing material may also comprise salts and/or sugars. In certain embodiments, the stabilizing materials may be substantially (including completely) cross-linked. The stabilizing material may be neutral, positively or negatively charged.

"Droplet" refers to a spherical or spheroidal entity which may be substantially liquid or which may comprise liquid and solid, solid and gas, liquid and gas, or liquid, solid and gas. Solid materials within a droplet may be, for example, particles, polymers, lipids, proteins, or surfactants.

"Vesicle" refers to an entity, which is generally characterized by the presence of one or more walls or membranes, which form one or more internal voids. Vesicles may be formulated, for example, from a stabilizing material such as a lipid, including the various lipids described herein, a proteinaceous material, including the various proteins described herein, and a polymeric material, including the various polymeric materials described herein. Vesicles may also be formulated from carbohydrates, surfactants, and other stabilizing materials, as desired. The lipids, proteins, polymers and/or other vesicle forming stabilizing materials may be natural, synthetic or semi-synthetic. Preferred vesicles are those, which comprise walls or membranes formulated from lipids. The walls or membranes may be concentric or otherwise. The stabilizing compounds may be in the form of one or more monolayers or bilayers. In the case of more than one monolayer or bilayer, the monolayers or bilayers may be concentric. Stabilizing compounds may be used to form a unilamellar vesicle (comprised of one monolayer or bilayer), an oligolamellar vesicle (comprised of about two or about three monolayers or bilayers) or a multilamellar vesicle (comprised of more than about three monolayers or bilayers). The walls or membranes of vesicles may be substantially solid (uniform), or they may be porous or semi-porous. The vesicles described herein include such entities commonly referred to as, for example, liposomes, lipospheres, particles, nanoparticles, micelles, bubbles, microbubbles, microspheres, lipid-coated bubbles, polymer-coated bubbles and/or protein-coated bubbles, microbubbles and/or microspheres, nanospheres, microballoons, microcapsules, aerogels, clathrate bound vesicles, hexagonal H II phase structures, and the like. The internal void of the vesicles may be filled with a wide variety of materials including, for example, water, oil, gases, gaseous precursors, liquids, stabilizing materials along with the therapeutic drug, and/or biological agents. The vesicles may also comprise a targeting ligand, if desired.

"Liposome" refers to a generally spherical or spheroidal cluster or aggregate of amphipathic compounds, including lipid compounds, typically in the form of one or more concentric layers, for example, bilayers. They may also be referred to herein as lipid vesicles. The liposomes may be formulated, for example, from ionic lipids and/or non-ionic lipids. Liposomes formulated from non-ionic lipids may be referred to as niosomes.

"Liposphere" refers to an entity comprising a liquid or solid oil surrounded by one or more walls or membranes.

"Micelle" refers to colloidal entities formulated from lipids. In certain preferred embodiments, the micelles comprise a monolayer, bilayer, or hexagonal H II phase structure.

"Clathrate" refers to a solid, semi-porous or porous particle, which may be associated with vesicles. In a preferred form, the clathrates may form a cage-like structure containing cavities, which comprise one, or more vesicles bound to the clathrate, if desired. A stabilizing material may, if desired, be associated with the clathrate to promote the association of the vesicle with the clathrate. Clathrates may be formulated from, for example, porous apatites, such as calcium hydroxyapatite, and precipitates of polymers and metal ions, such as alginic acid precipitated with calcium salts.

"Antibubble" refers to a composition having a central sphere of lipid surrounded by a gas, liquid, or gas/liquid mixture, such as, for example, a perfluorocarbon, which in turn is surrounded by a stabilizing material, such as, for example, a surfactant or oil. One or more targeting ligands may be incorporated into the surface of the antibubble.

"Gas filled vesicle" refers to a vesicle having a gas encapsulated therein. "Gaseous precursor filled vesicle" refers to a vesicle having a gaseous precursor encapsulated therein. The vesicles may be minimally, partially, substantially, or completely filled with the gas and/or gaseous precursor.

"Host" or "patient" refers to animals, including mammals, preferably humans.

"Region of a patient" refers to a particular area or portion of the patient and in some instances to regions throughout the entire patient. Exemplary of such regions include specific tissues, organs, organ systems, cells, receptors and intracellular compartments. The "region of a patient" is preferably internal, although, if desired, it may be external.

"Region to be targeted" or "targeted region" refer to a region of a patient where delivery of a therapeutic is desired. "Region to be imaged" or "imaging region" denotes a region of a patient where diagnostic imaging is desired.

"Therapeutic" refers to any pharmaceutical, drug or prophylactic agent, which may be used in the treatment (including the prevention, diagnosis, alleviation, or cure) of a malady, affliction, disease or injury in a patient. Therapeutic includes contrast agents and dyes for visualization. Therapeutically useful peptides, polypeptides and polynucleotides may be included within the meaning of the term pharmaceutical or drug.

"Genetic material" refers generally to nucleotides and polynucleotides, including deoxyribonucleic acid (DNA) and ribonucleic acid (RNA). The genetic material may be made by synthetic chemical methodology known to one of ordinary skill in the art, or by the use of recombinant technology, or by a combination thereof. The DNA and RNA may optionally comprise unnatural nucleotides and may be single or double stranded. "Genetic material" also refers to sense and anti-sense DNA and RNA, that is, a nucleotide sequence, which is complementary to a specific sequence of nucleotides in DNA and/or RNA.

"Targeting ligand" refers to any material or substance, which may promote targeting of tissues and/or receptors in vivo or tit vitro with the compositions of the present invention. The targeting ligand may be synthetic, semi-synthetic, or naturally-occurring. Materials or substances which may serve as targeting ligands include, for example, proteins, including antibodies, antibody fragments, hormones, hormone analogues, glycoproteins and lectins, peptides, polypeptides, amino acids, sugars, saccharides, including monosaccharides and polysaccharides, carbohydrates, vitamins, steroids, steroid analogs, hormones, cofactors, and genetic material, including nucleosides, nucleotides, nucleotide acid constructs and polynucleotides.

"Diagnostic agent" refers to any agent, which may be used in connection with methods for imaging an internal region of a patient and/or diagnosing the presence or absence of a disease in a patient. Exemplary diagnostic agents include, for example, contrast agents for use in connection with ultrasound imaging, magnetic resonance imaging or computed tomography imaging of a patient. Diagnostic agents may also include any other agents useful in facilitating diagnosis of a disease or other condition in a patient, whether or not imaging methodology is employed.

"Cross-link," "cross-linked" and "cross-linking" generally refer to the linking of two or more stabilizing materials, including lipid, protein, polymer, carbohydrate, surfactant stabilizing materials and/or bioactive agents, by one ore more bridges. The bridges may be composed of one or more elements, groups, or compounds, and generally serve to join an atom from a first stabilizing material molecule to an atom of a second stabilizing material molecule. The cross-link bridges may involve covalent and/or non-covalent associations. Any of a variety of elements, groups, and/or compounds may form the bridges in the cross-links, and the stabilizing materials may be cross-linked naturally or through synthetic means. For example, cross-linking may occur in nature in material formulated from peptide chains, which are joined by disulfide bonds of cystine residues, as in keratins, insulins and other proteins. Alternatively, cross-linking may be effected by suitable chemical modification, such as, for example, by combining a compound, such as a stabilizing material, and a chemical substance that may serve as a cross-linking agent, which may cause to react by, for example, exposure to heat, high-energy radiation, ultrasonic radiation and the like. Examples include cross-linking by sulfur to form disulfide linkages, cross-linking using organic peroxides, cross-linking of unsaturated materials by means of high-energy radiation, cross-linking with dimethylol carbamate, and the like. If desired, the stabilizing compounds and/or bioactive agents may be substantially cross-linked.

"Biocompatible" refers to materials which are generally not injurious to biological functions and which will not result in any degree of unacceptable toxicity, including allergenic responses and disease states.

"In combination with" refers to the incorporation of bioactive agents, drugs prodrugs, and/or targeting ligands, in a composition of the present invention, including emulsions, suspensions and vesicles. The drug, prodrug, bioactive agent and/or targeting ligand can be combined with the therapeutic delivery system and/or stabilizing compositions (including vesicles) in any of a variety of ways. For example, they may be associated covalently and/or non-covalently with the delivery system or stabilizing materials. Alternatively the drug or agent may be entrapped within the internal void(s) of the delivery system or vesicle. The drug or agent may also be integrated within the layer(s) or wall(s) of the delivery system or vesicle. The drug or agent may also be located on the surface of a delivery system or vesicle or non-vesicular stabilizing material. The drug or agent may interact chemically with the walls of the delivery system including, for example, the inner and/or outer surfaces of the delivery system, vesicle and may remain substantially adhered thereto.

"Tissue" refers generally to specialized cells, which may perform a particular function. The term "tissue" may refer to an individual cell or a plurality or aggregate of cells, for example, membranes, blood or organs.

"Receptor" refers to a molecular structure within a cell or on the surface of a cell, which is generally characterized by the selective binding of a specific substance.

"Intracellular" or "intracellularly" refers to the area within the plasma membrane of a cell, including the protoplasm, cytoplasm and/or nucleoplasm. "Intracellular delivery" refers to the delivery of a bioactive agent, such as a targeting ligand and/or steroid prodrug, into the area within the plasma membrane of a cell.

"Cell" refers to any one of the minute protoplasmic masses, which make up organized tissue, comprising a mass of protoplasm surrounded by a membrane, including nucleated and unnucleated cells and organelles.

"In vivo delivery" refers to delivery of a biologic by such routes of administration as oral, intravenous, subcutaneous, intraperitoneal, intrathecal, intramuscular, intracranial, inhalational, topical, transdermal, suppository (rectal), pessary (vaginal), and the like.

### II. Cavitation

The acoustic waves generated by the above mentioned acoustic wave generators have compressive and/or tensile components with a specific peak compressive and tensile pressure.

In the preferred embodiment the tensile wave component is produced first and then followed by the high pressure component to gain a maximum possible cavitation. This is in contrary to the conventional medical shock waves as they are generated with low tensile wave components in order to minimize the cavitational effects. However, conventional medical shock waves are used in an alternative version of the preferred embodiment for combining cavitation induced sonoluminescence with high pressure effects ofthe shock waves.

For inducing cavitation inside a fluid medium acoustic waves are used which have typically a rarefaction phase or a tensile wave component with about - 10 MPa in water, but it varies in accordance to the used medium in the range of - 5 MPa to - 15 MPa. Using specific conditions in the medium it may be also below or above this specified region.

During the cavitation bubbles are generated inside the medium. These bubbles collapse within a certain time after the tensile wave component has passed the region, where the bubbles have been generated by the acoustic wave. These collapsing bubbles emit light pulses, which is also called sonoluminescence. These light pulses emitted by the collapsing bubbles are ultra short light pulses, which are considered to be especially reactive to molecular bonds.

The light pulses produced by the cavitation or sonoluminescence in the medium are ultra short light pulses. The duration of these light pulses is considered to be in the range of several hundred femtoseconds to several hundred picoseconds.

As above mentioned, ions or radicals are generated inside a cavitation bubble during the expansion and collapse of the bubbles.

In a further embodiment, these ions or radicals generated inside the cavitation bubbles are also used for assisting or catalyzing the activating or deactivating of chemical reactions in combination with the light generated by the sonoluminescence. The chemical reactions are preferably limited to these ones, which are sensitive to electromagnetic excitation. However, as above mentioned these chemical reactions are also sensitive to combinations of electromagnetic excitation within the IR, visible or UV range, shock wave induced effects and chemical or polarization effects of the ions or radicals.

The size of the bubbles and the collapse time varies wit the tensile pressure of the acoustic wave. Therefore, by variation of the tensile pressure and/or the ratio of the tensile to the compressive pressure the size of the bubbles and the collapse time is controllable. Furthermore, this provides influence to the spectrum of the light pulses emitted by the sonoluminescence.

Additionally, the spectrum of the sonoluminescence is changeable by the gas content of the cavitation bubbles, thus influencing the effectiveness of the activation or deactivation of the chemical reactions by the spectrum of the emitted light pulses.

In one alternative of the preferred embodiment the spectral content of the cavitation collapse light is changed by supplying an appropriate gas inside the compound or substance that enhances the cavitation process. In another alternative of the preferred embodiment the spectral content of the cavitation collapse light is changed by adding an appropriate gas to the normal breathing gas of the host.

The inventors of the present invention have unexpectedly found that the generation of cavitation by an acoustic device at a peak tensile pressure of -5 MPa to -15 MPa allows the initiation or deactivation of chemical reactions via electromagnetic radiation within the infrared, visible or ultraviolet range, and that these chemical reactions can be initiated or deactivated using this technique without being limited by the absorption or penetration depth of the electromagnetic wave inside the host. For inducing cavitation inside a host, conventional ultrasonic devices or shock wave devices have been used which have a rarefaction phase or a tensile wave component with about - 10 MPa in water. However, conventional shock wave devices are designed in such a way that the tensile wave component is as small as possible in order to avoid the cavitation. In contrast, the inventors of the present invention have discovered that when a maximal cavitation effect is produced, sonoluminescence, occurs in which the tensile wave component is produced first followed by the high pressure component. This is the opposite effect of a conventional shock wave, where the high pressure part comes first followed by the tensile wave component. The inventors have found that the optimal effects occur at peak tensile pressure of these acoustic waves in the range of -5 MPa to -15 MPa. They have discovered that the invention disclosed herein combines the processes of electromagnetic excitation of molecules for targeted initiation or deactivation of chemical reactions together with a shock wave induced permeability of a cell membrane.

The invention provides an apparatus and method which produces acoustic waves with such an amplitude that cavitation is induced inside the host wherein due to the cavitation collapsing bubbles are generated which emit both shock waves and electromagnetic radiation within the infrared, visible and ultraviolet range and wherein the thus generated light is used for initiating or deactivating chemical reactions inside the host or body. A significant advantage to this is that due to the acoustic wave induced sonoluminescence, the light can be generated within a focus of a shock wave apparatus nearly everywhere inside a host without the necessity of penetrating the host with a catheter or optical fiber. An additional advantage is that the light pulses produced by cavitation or sonoluminescence are ultra short light pulses, which are considered especially influential in breaking or forming molecular bonds. Moreover, as the acoustic or shock waves can be focused on a small area the cavitation and therefore the sonoluminescence is restricted to a limited region for the desired therapy.

The amplitudes of the tensile waves produced by the collapse of the cavitation bubbles are also sufficient to produce disruption of a material that is encapsulating a prodrug, drug or biological agent. Such an encapsulation material can include but is not limited to microspheres, micelles, liposomes, dendrimers, clathrates and the like. Thus, in one embodiment of the present invention, the electromagnetic excitation can be used to activate for example, a prodrug via sonoluminescence followed by disruption of the encapsulation material via shockwave induced shear stress.

As one skilled in the art will recognize, in order to achieve a maximum efficiency of these processes the acoustic waves can be designed with respect to whether the compressive or the tensile part component is initiated first or with respect to the magnitude and/or ratio of the compressive to tensile pressure.

Cavitation, among the non-thermal effects of energetic beams, e.g., ultrasound, has the greatest potential for therapeutic applications, when controlled. The present invention exploits selective targeting of tissue based on cavitation. In some embodiments, it may be desirable that two different energy frequencies are crossed within tissue with the overlap region generating a difference frequency (beat frequency) where the two beams cross. Cavitation, e.g., ultrasonic waves or hyperthermia, e.g., ultrasonic waves and light waves, e.g., laser, occur at the point of intersection of the two beams. See for example U.S. Patent No. 6,428,532 to Doukas et al. The method takes advantage of the fact that the cavitation threshold and/or hyperthermia depends on the frequency of the energetic beam, e.g., laser or ultrasound. For example, low frequency ultrasound is more effective in producing cavitation than high frequency ultrasound. The frequencies of the two ultrasound beams are set above the cavitation threshold. The two beams are made to overlap spatially and temporally inside the target to produce cavitation. For example, if the frequency of two ultrasound beams are 4.0 and 3.8 MHZ, respectively, 200-kHz ultrasound is generated at the overlap region which is more effective in producing cavitation than either the 4.0-MHZ or the 3.8-MHZ ultrasound. Thus, only at the overlap region of the two ultrasound beams and nowhere else in the tissue, will cavitation be produced.

Ultrasound-induced cavitation is responsible for the permeabilization of the cell plasma membrane. The membrane permeabilization is transient and the plasma membrane recovers. The permeabilization of the plasma membrane allows large molecules to diffuse into the cytoplasm. Localized generation of low frequency ultrasound can also be applied to selective sites of an organ or tissue for drug delivery or as a vector for gene therapy.

"Cavitation seeds" are compounds or substances that are introduced, for example, in the form of gas-filled microspheres, e.g., liposomes. In one aspect, the prodrug, drug or biological agent to be delivered functions as a cavitation seed. Alternatively, other compounds or substances that enhance the cavitation process can be included as part of the chemical reaction or be delivered with the drug or biologic agent. Besides, gas filled microspheres, such as a liposomes, other such compounds or substances include but are not limited to, ultrasonic contrast agents, any inorganic gas filled molecule that does not reflect, any material with a low acoustic impedance such as a plastic bead, a lipid or oil droplet, and any material with a high acoustic impedance including an inert material such as small gold pellets. Since acoustic contrast agents essentially contain stable air bubbles. They enhance the echo in diagnostic applications of ultrasound. Bubble-based contrast agents have been shown to enhance cavitation during ultrasound exposure. Acoustic contrast agents can also be used to increase the efficiency of cavitation and provide an additional degree of selectivity. Suitable contrast agent include, for example, Albunex-TM. (Molecular Biosystems, San Diego and Levovist-TM.) (Schering AG, Berlin, Germany). Albunex-TM. is a coated microbubble produced by sonication of an albumin solution. Levovist-TM. is made of dry particles of galactose which form a microbubble suspension when water is added.

The spectral content of the light pulse may be determined by the mixture of gas inside the bubbles. Further, in a clinical setting, a patient can be allowed to breathe an appropriate gas which would then change the spectral content of collapsing cavitation bubble light. Cavitation seeds are also generated by acoustic pulses or pulse sequences, as well as continuous ultrasound of sufficient amplitude.

A distinct advantage of the current invention is that it has been discovered that unlike methods to induce cavitation know in the prior art, it is not necessary to use shockwaves to achieve the desired cavitational action. The inventors have unexpectedly discovered that it is sufficient to exceed the cavitation threshold in the fluid by applying ultrasound pulses with a rarefaction phase of -5 to -15 MPa. The optimal frequency is chosen based upon the type of energy source used that will produce pulses at that cavitation threshold of -5 to 15 MPa. In a preferred embodiment, acoustic waves are generated of a sufficient amplitude at a peak tensile pressure of -12 MPa to induce cavitation. This is in contrast to traditional diagnostic ultrasound applications which typically produce peak negative pressures in the range of 0.1-3.5 MPa occurring at lower frequencies. Cavitation produced at lower thresholds is capable of only inducing shock waves.

### III. Acoustic Energy Source

The acoustic energy source of the current invention may be of any appropriate design recognized by those skilled in the art, i.e. ultrasound, capable of producing pulses with a rarefaction phase of -5 MPa to -15 MPa in water. Preferably, acoustic waves are generated of a sufficient amplitude at a peak tensile pressure of -12 MPa to induce cavitation. In some embodiments the acoustic device is external, the energy being applied directly to the desired region of the host. In other embodiments described herein, the energy is utilized externally to a fluid-filled chamber. In yet other embodiments, the acoustic energy is applied internally to the host, the device being inserted by any available means familiar to one skilled in the art. As a non-limiting example, the acoustic device is miniaturized and is inserted by means of a cannulae into a vein or artery. In other embodiments, the acoustic device is inserted orally, anally or vaginally. In yet other embodiments, it is contemplated that the device may be surgically implanted directly into the host and then operated at times when treatment or drug delivery is necessary.

As non-limiting examples, ultrasound may vary in frequency between about 1 KHz and 100 MHz. For the current invention, it is desired to select an optimal frequency that will produce pulses at that cavitation threshold of -5 to -12 MPa. The therapeutic frequency and imaging frequencies may be the same or may be swept. Imaging and therapeutic frequencies and imaging and therapeutic energies may each be the same or different. Most preferably a single frequency pulse or series of pulses (train of continuous wave pulses) is applied to the host. Alternatively, a burst of pulses is employed. Superimposition of first and second frequencies results enhancing bubble rupture and local drug delivery. As one skilled in the art would recognize, however, the level of peak energy which is selected will vary depending upon the specific application, the duty cycle, pulse repetition rate, frequency and other factors. In general the requisite amount of therapeutic ultrasound energy may vary approximately by the reciprocal of the square root of the frequency.

As one skilled in the art will readily appreciate, any type of imaging technique can be employed in combination with, or used prior to the application of, the acoustic energy of the current invention. This may be done, for example, to visualize the exact location of the tumor to be treated or the diseased or affected area to receive therapy.

The choice of the optimal frequency to produce the desired cavitational threshold pressure of -5MPa to -15 MPa will depend upon a number of factors as one skilled in the art will recognize. In principle, the carrier frequency, the duration of the energy pulse signal the essential factors to reach the desired amplitude and duration of the negative pressure phase which is the cavitation threshold. One skilled in the art will recognize that these factors will also vary depending upon the tissue being targeted. For example higher frequencies provide higher spatial resolution for imaging and also higher spatial localization for therapy.

Any of the encapsulated drugs or biological agents or any enzymatic reaction targeted by the current invention may be activated by acoustic energy for localized drug delivery. The therapeutic delivery systems of the invention, however, are also competent drug delivery vehicles without the administration of ultrasound. For example, therapeutic delivery systems comprising targeting ligands may fuse to cells within the body so that they may be activated via the cavitation processes taken advantage of by the current invention.

The delivery of drugs or biological agents using acoustic energy is best accomplished for tissues which have a good acoustic window for the transmission of the energy. This is the case for most tissues in the body such as muscle, the heart, the liver and most other vital structures. In the brain, in order to direct the energy past the skull a surgical window may be necessary.

It is a further embodiment of this invention in which sound activation affords site specific delivery of the drug or agent. In one aspect, the gas and/or gaseous precursor containing vehicles are echogenic and visible on ultrasound. Ultrasound can be used to image the target tissue and to monitor the drug carrying vehicles as they pass through the treatment region. As increasing levels of ultrasound are applied to the treatment region, this breaks apart the delivery vehicles and/or releases the drug within the treatment region. Release of the drug is generally meant to include either release of the drug or agent from the delivery vehicle but not from a linking group or release from the covalently bonded lipid moiety and/or the linking group, but not from the delivery vehicle. Alternatively, release of the drug from both the delivery vehicle and from the covalently bonded lipid moiety and/or the linking group may be preferable.

Release and/or vesicle rupture can be monitored ultrasonically by several different mechanisms. Bubble or vesicle destruction results in the eventual dissolution of the ultrasound signal. However, prior to signal dissolution, the delivery vehicles/vesicles provide an initial burst of signal. Therefore, as increasing levels of ultrasound energy are applied to the treatment zone containing the delivery vehicles/vesicles, there is a transient increase in signal. This transient increase in signal may be recorded at the fundamental frequency, the harmonic, odd harmonic or ultraharmonic frequency.

In the case of diagnostic applications, such as ultrasound and CT, energy, such as ultrasonic energy, is applied to at least a portion of the patient to image the target tissue. A visible image of an internal region of the patient is then obtained, such that the presence or absence of diseased tissue can be ascertained. With respect to ultrasound, ultrasonic imaging techniques, including second harmonic imaging, and gated imaging, are well known in the art, and are described, for example, in Uhlendorf, IEEE Transactions on Ultrasonics, Ferroelectrics, and Frequency Control, 14(1):70-79 (1994) and Sutherland, et al., Journal of the American Society of Echocardiography, 7(5):441-458 (1994). CT imaging techniques which are employed are conventional and well known to those skilled in the art. See for example, Computed Body Tomography, Lee, Sagel, and Stanley, eds., 1983, Ravens Press, New York, N.Y.

Alternatively, it may be advantageous to utilize higher energy sources depending upon the region within the host to be targeted. For example, areas of the body which are generally characterized by larger amounts of muscle mass, as well as highly vascularized tissues, such as heart tissue, may require a larger duty cycle, for example, up to about 100%.

One skilled in the art will also recognize that an acoustic device may be used which employs two frequencies of ultrasound. The first frequency may be x, and the second frequency may be 2 times. In one embodiment, the device can be designed such that the focal zones of the first and second frequencies converge to a single focal zone. The focal zone of the device may then be directed to the targeted compositions, for example, targeted vesicle compositions, within the targeted tissue. This device may provide second harmonic therapy with simultaneous application of the x and 2 times the frequencies of ultrasound energy. It is contemplated that, in the case of encapsulated drugs or biological agents, this second harmonic therapy may provide improved rupturing of vesicles as compared to the energy involving a single frequency. Also, it is contemplated that the preferred frequency range may reside within the fundamental harmonic frequencies of the encapsulating material. Lower energy may also be used with this device. A device which may be employed in connection with the aforementioned second harmonic therapy is described, for example, in Kawabata, et al., Ultrasonics Sonochemistry, 3:1-5 (1996).

For therapeutic drug delivery, the rupturing of the bioactive agent containing the targeted drug or biological agent of the invention is surprisingly easily carried out by applying acoustic energy of a certain frequency to the region of the patient where therapy is desired, after the encapsulated therapeutic have been administered to or have otherwise reached that region, e.g., via delivery with targeting ligands.

A variety of types of diagnostic acoustical devices may be employed in the practice of the invention, the particular type or model of the device not being critical to the method of the invention. Also suitable are devices designed for administering ultrasonic hyperthermia, such devices being described in U.S. Pat. Nos. 4,620,546, 4,658,828, and 4,586,512. The device may optionally employ a resonant frequency (RF) spectral analyzer. As discussed herein, probes may be applied externally or may be implanted Typically, regardless of the device employed, in order to utilize the phenomenon of cavitation to release the therapeutic agent and/or activate a particular chemical reaction within or external to the host, frequency energies are optimized for the generation of the acoustic waves at a peak tensile pressure of -5 MPa to -15 MPa to induce cavitation.

For treating a particular type of tissue, in a predetermined part of the body, the selection of the parameters should be made in the view of clinical experience. For example, the object is to acoustically generate cavitation bubbles in the proliferating cell area to cause mechanically induced significant alterations in the tissue environs and/or alterations in the tissue cells themselves. Such alterations are indicated by a fall in, an obstruction of, or even an interruption of, the blood supply to a cell, as well as by acoustically induced interference with the existing mode of nutrition of the cell, and/or by destruction of the cell elements (rips in the cell membranes, destruction of elements of the cell contents), but not necessarily by destruction of the structural cohesion of the cellaggregate.

In still a further embodiment of the present invention the apparatus for targeted activation or deactivation of chemical reactions further comprising manipulating means for manipulating a spectrum of said photons generated by said controlled cavitation processes. As above mentioned this is achieved e.g. by influencing the bubble collapse time, the bubble radius or the gas inside the bubble.

This apparatus or method is not restricted to breaking up molecular bonds but also includes other chemical reactions like folding of molecules or combining molecules or combining molecules with one or more ions or radicals. Additionally, this apparatus or method is not restricted to target cells, a system of cells or a tissue which is penetrated by molecules due to a transient permeabilization of the cell membranes by the cavitation effects, but it may be used for any light induced chemical reactions within a specific region of the host or body.

In still a further embodiment induced chemical reactions are limited to chemical bonds or molecular bonds, which are especially sensitive at a special excitation wavelength or which may be activated or deactivated in a particular effective way. This is of particular importance in the case when broken molecular bonds or broken chemical bridges may cause toxic reactions.

As already mentioned the cavitation is used on the one hand for generating light pulses and on the other hand for enabling transient permeabilization of the cell membrane nearly simultaneously. Using both effects in combination is a further embodiment of the present invention.

To combine the processes of electromagnetic excitation of molecules for targeted initiation or deactivation of chemical reactions together with a shock wave induced permeability of a membrane and in order to achieve a maximum efficiency of these processes the acoustic waves may be designed in respect to whether the compressive or the tensile part comes first and/or in the respect to the magnitude and/or ratio of the compressive to tensile pressure.

Therefore, these amplitudes are also sufficient to produce disruption of a microsphere. However, this is only one alternative embodiment, where the electromagnetic excitation of molecules via sonoluminescence and disruption of a microsphere via shockwave induces shear stress. Therefore, the disruption of a microsphere by compression and expansion via a shock wave for releasing e.g. prodrugs, which are activated or deactivated via the sonoluminescence, is considered as an additional step of the preferred embodiment of the present invention.

Examples for molecular bonds which are sensitive to ultra short light pulses within the IR, visible or UV range are intermolecular or intramolecular hydrogen bonds. However, other intermolecular or intramolecular bonds, which are sensitive to an electromagnetic excitation within the spectrum of the sonoluminescence, may be target bonds for the preferred embodiment.

Generally it is important that at a specific time a minimum concentration of both substances exists at a specific region. Therefore any order of delivery of the substances is possible as long as the above-mentioned minimum concentration of both substances for initiating cavitation and for initiating the chemical reactions is fulfilled.

For the delivery of the chemical reactive substances the processes known from the pharmacokinetic prior art can be used for the delivery of the substances. Two typical examples are the systematic injection and the other one is the local injection. However, other pharmacokinetic processes can be used for the delivery of the drugs or pre-drugs. The cavitation seeds and chemical reactive substances are transportable in different microspheres not only with the same pharmacokinetic process but also with different pharmacokinetic processes to prevent an unwanted chemical reaction of both substances outside of the therapy region.

In another preferred embodiment of the present invention the chemical substances are activated or deactivated via light impulses applied to the chemical substances. However, the chemical substances may be transported to the therapy region within microspheres.

Here it has to be considered that cavitation seeds and chemical reactive substances may be delivered separately or as a combination. Therefore, there is the first alternative that the caviation seeds are one substance and the pre-drug or drug is a second substance. The second alternative is that the chemical reactive substances are the cavitation seeds themselves. The third alternative is that the cavitation seeds contain the chemical reactive substances, which are released during the cavitation process from the cavitation seed. Further alternatives or combinations are possible.

### IV. Encapsulation of the Drug or Agent

Entrapping the drug or biological agent inside a host molecule, or producing the chemotherapy agent in situ by fragmentation of a large, essentially inert molecule, improves the effectiveness of existing drug therapy agents. The drug or biological agent is trapped in a host molecule which is inert to bodily fluids. The combined structure is then injected into the patient. The confined agent distributes itself innocuously throughout the body, with at most a very slow diffusion of the agent from the host molecule. At the desired location, external or internal acoustical excitation fragments the bonds of the external structure of the host molecule and the active drug or biological agent is released.

The drugs or biological agents used in the current invention can be encapsulated by any of a number of means known to those skilled in the art. Thus drugs or biological agents can be delivered in several ways, including but not limited to, gas-filled microspheres, clathrate compounds, polymer shells, liposomes and dendrimer compounds. See for example, US. Patent Nos. 6,444,217 to Kwok et al; 6,465,006 to Zhang et al; 6,443,898 and. 6,461,586 to Under et al; 5,498,421, 5,635,207, 5,639,473, 5,650,156, 5,665,382 and 5,665,383 to Grinstaff et al; 5,531,980, 5,567,414, 5,543,553, and 5,658,551 to Schneider et al; and 5,795,581 to Segalman et al.

Whichever method is chosen requires that the bonds that comprise the encapsulating material are able to be broken by the agitation caused by the cavitation bubbles following the application of the acoustical energy either from an external source or one internal to the host, for example within a blood vessel.

### Dendrimers

A dendrimer is a polymer or co-polymer comprising multiple branched chains attached at the bases. Thus, a dendrimer is a highly branched polymer, usually roughly spherical in shape, where the polymer chains are linked to a central core. If the branching rate increases faster than the surface area of the dendrimer as the dendrimer grows in size, steric hindrance between the densely packed branch ends at the surface eventually forces an overall structure having flexible internal cavities. Their synthesis is described in detail in U.S. Patent No. 5,795,581 to Segalman et al. The combination of steric hindrance and conformational constraints of the molecules comprising the dendrimer creates spaces within the structure of the dendrimer in which drug or biological agent molecules can reside, held in place without chemical binding between the dendrimer and the particular molecule. Drug molecules can be introduced into these by a variety of means including diffusion, usually with the aid of high pressure. This works well for small molecules. Electrical attraction between polar regions of the dendrimer and the drug molecule can also be taken advantage of to place the drug within the dendrimer. One so skilled in the art will also recognize that the dendrimer can be synthesized in the presence of the drug molecule, thereby entrapping the drug molecule during the process of growth of the dendrimer. Thus, the dendrimers are synthesized in a solution containing the drug molecule as a constituent. As the guest molecule is chemically substantially inert with respect to the dendrimer, it does not participate in the dendrimer growth. However, as the dendrimer continues to grow and the internal cavities gain definition, a drug molecule can become mechanically trapped.

When energy is applied to the dendrimer core molecule, the bond length at the core may increase, thus causing an increase in the dendrimer diameter, thereby relieving the steric forces at the surface of the dendrimer, and hence opening channels for escape of the drug molecules. Alternatively, agitation by the cavitation bubble may lead to a breaking a core molecule bond or some other critical branch point in the dendrimer core, and thus release of one of the dendrimer wedges. The drug molecules can easily escape from the resulting structure. Thus the introduction of acoustic energy will allow easily delivery of the drug molecule from the core.

### Polymer Shells

Another useful composition for the targeted delivery of drugs or biological agents is a polymeric shell. The polymeric shell is a biocompatible material, cross-linked by the presence of disulfide bonds. The polymeric shell associated with the drug or biological agent is optionally suspended in a biocompatible medium for administration. The delivery of drugs and biological agents in the form of a microparticulate suspension within a polymeric shell allows targeting to organs through the use of particles of varying size, and through administration by different routes. One skilled in the art would further recognize that using polymeric shells, one can deliver substantially water insoluble pharmacologically active agents, employing a much smaller volume of liquid and requiring greatly reduced administration time relative to administration volumes and times. Delivery of the acoustic energy at the desired site of action of the drug allows precise delivery of the drug or biological agent so encapsulated.

The drug or biological agent is optionally dispersed within a solid, a liquid or a gas within the polymeric shell. One skilled in the art can easily recognize that one or more drugs or biological agents can be so delivered. Typically, the largest cross-sectional dimension of a shell is no greater than about 10 microns and the polymeric shell will comprise a biocompatible material which is substantially crosslinked by way of disulfide bonds. The exterior of the polymeric shell can be optionally modified by a drug or biological agent (e.g. a receptor molecule or antibody or antibody fragment) linked to the shell through a covalent linkage.

A number of biocompatible materials may be used to form a polymeric shell. Essentially any material, natural or synthetic, bearing sulfhydryl groups or disulfide bonds within its structure may be utilized for the preparation of a disulfide crosslinked shell. The sulfhydryl groups or disulfide linkages may be preexisting within the structure of the biocompatible material, or they may be introduced by a suitable chemical modification. For example, naturally occurring biocompatible materials such as proteins, polypeptides, oligopeptides, polynucleotides, polysaccharides (e.g., starch, cellulose, dextrans, alginates, chitosan, pectin, hyaluronic acid, and the like), lipids, and so on, are candidates for such modification. Other linkages, such as esters, amides, ethers, and the like, can also be formed during the ultrasonic irradiation step (so long as the requisite functional groups are present on the starting material). One skilled in the art would easily recognize other examples of suitable biocompatible materials including, but limited to, naturally occurring or synthetic proteins, so long as such proteins have sufficient sulfhydryl or disulfide groups so that crosslinking (through disulfide bond formation, for example, as a result of oxidation during ultrasonic irradiation) can occur. Examples of suitable proteins include albumin (which contains 35 cysteine residues), insulin (which contains 6 cysteines), hemoglobin, lysozyme (which contains 8 cysteine residues), immunoglobulins, alpha-2-macroglobulin, fibronectin, vitronectin, fibrinogen, and the like, as well as combinations of any two or more thereof.

Other functional proteins, such as antibodies or enzymes, which could facilitate targeting of biologic to a desired site, can also be used in the formation of the polymeric shell. Similarly, synthetic polypeptides containing sulfhydryl or disulfide groups are also good candidates for formation of particles having a polymeric shell. In addition, polyalkylene glycols (e.g., linear or branched chain), polyvinyl alcohol, polyhydroxyethyl methacrylate, polyacrylic acid, polyethyloxazoline, polyacrylamide, polyvinyl pyrrolidinone, and the like, are good candidates for chemical modification (to introduce sulfhydryl and/or disulfide linkages) and shell formation (by causing the crosslinking thereof).

The drug or biological agent typically is suspended or dissolved in a dispersing agent within the polymeric shell. Dispersing agents include any liquid that is capable of suspending or dissolving biologic, but does not chemically react with either the polymer employed to produce the shell, or the biologic itself. Examples include water, vegetable oils, aliphatic, cycloaliphatic, or aromatic hydrocarbons having 4-30 carbon atoms, aliphatic or aromatic alcohols having 1-30 carbon atoms (e.g., octanol, and the like), aliphatic or aromatic esters having 2-30 carbon atoms (e.g., ethyl caprylate (octanoate), and the like), alkyl, aryl, or cyclic ethers having 2-30 carbon atoms (e.g., diethyl ether, tetrahydrofuran, and the like), alkyl or aryl halides having 1-30 carbon atoms (and optionally more than one halogen substituent, e.g., CH₃Cl, CH₂Cl₂, CH₂Cl―CH₂Cl, and the like), ketones having 3-30 carbon atoms (e.g., acetone, methyl ethyl ketone, and the like), polyalkylene glycols (e.g., polyethylene glycol, and the like), or combinations of any two or more thereof.

Other combinations of dispersing agents include volatile liquids such as dichloromethane, ethyl acetate, benzene, and the like (i.e., solvents that have a high degree of solubility for the pharmacologically active agent, and are soluble in the other dispersing agent employed), along with a less volatile dispersing agent. When added to the other dispersing agent, these volatile additives help to drive the solubility of the pharmacologically active agent into the dispersing agent.

Once injected into the host, the polymeric shell containing the drug or biological agent is subjected to the acoustal energy capable of initiating the cavitation process at the site of desired action of the drug or agent. The energy is applied under conditions and for a time sufficient to promote the break-up of the crosslinking of the biocompatible material of the disulfide bonds by the cavitation process.

### Liposomes and Microspheres

Laminarized surfactants in the form of liposomes and gas-filled micrspheres are also useful to encapsulate drugs or biological agents in the practice of the current invention. Suspensions are obtained by exposing the laminarized surfactants to air or a gas before or after admixing with an aqueous phase. Such microbubles can be manufactured to allow dispersion within the host and the desired location using the acoustic means disclosed herein. A wide variety of materials can be used as liquids, gases and gaseous precursors for entrapping within the carriers. For gaseous precursors, it is only required that the material be capable of undergoing a phase transition to the gas phase upon passing through the appropriate temperature. Gases useful in the include, but are not limited to, hexafluoroacetone, isopropyl acetylene, allene, tetrafluoroallene, boron trifluoride, 1,2-butadiene, 2,3-butadiene, 1,3-butadiene, 1,2,3-trichloro-2-fluoro-1,3-butadiene, 2-methyl-1,3-butadiene, hexafluoro-1,3-butadiene, butadiene, 1-fluorobutane, 2-methylbutane, perfluorobutane, decafluorobutane, 1-butene, 2-butene, 2-methyl-1-butene, 3-methyl-1-butene, perfluoro-1-butene, perfluoro-2-butene, 4-phenyl-3-butene-2-one, 2-methyl-1-butene-3-yne, butyl nitrate, 1-butyne, 2-butyne, 2-chloro-1,1,1,4,4,4-hexafluorobutyne, 3-methyl-1-butyne, perfluoro-2-butyne, 2-bromobutyraldehyde, carbonyl sulfide, crotononitrile, cyclobutane, methylcyclobutane, octafluorocyclobutane, perfluorocyclobutene, 3-chlorocyclopentene, perfluorocyclopentane, octafluorocyclopentene, cyclopropane, perfluorocyclopropane, 1,2-dimethylcyclopropane, 1,1-dimethylcyclopropane, 1,2-dimethylcyclopropane, ethylcyclo-propane, methylcyclopropane, diacetylene, 3-ethyl-3-methyl diaziridine, 1,1,1-trifluoro-diazoethane, dimethylamine, hexafluorodimethylamine, dimethylethylamine, bis(dimethyl-phosphine)amine, perfluoroethane, perfluoropropane, perfluoropentane, perfluorohexane, perfluoroheptane, perfluorooctane, perfluorononane, perfluorodecane, hexafluoroethane, hexafluoropropylene, octafluoropropane, octafluorocyclopentene, 1,1-dichlorofluoroethane, hexafluoro-2-butyne, octafluoro-2-butene, hexafluorobuta-1,3-diene, 2,3-dimethyl-2-norborane, perfluorodimethylamine, dimethyloxonium chloride, 1,3-dioxolane-2-one, 4-methyl-1,1,1,2-tetrafluoroethane, 1,1,1-trifluoroethane, 1,1,2,2-tetrafluoroethane, 1,1,2-trichloro-1,2,2-trifluoroethane, 1,1-dichloroethane, 1,1-dichloroethylene, 1,1-dichloro-1,2-difluoroethylene, 1,1-dichloro-1,2,2,2-tetrafluoroethane, 1,2-difluoroethane, 1-chloro-1,1,2,2,2-pentafluoroethane, 2-chloro-1,1-difluoroethane, 1,1-dichloro-2-fluoroethane, 1-chloro-1,1,2,2-tetrafluoroethane, 2-chloro-1,1-difluoroethane, chloroethane, chloropentafluoroethane, dichlorotrifluoroethane, fluoroethane, nitropenta-fluoroethane, nitrosopentafluoroethane, perfluoroethylamine, ethyl vinyl ether, 1,1-dichloroethane, 1,1-dichloro-1,2-difluoroethane, 1,2-difluoroethane, 1,2-difluoroethylene, methane, trifluoromethanesulfonylchloride, trifluoromethanesulfenylchloride, (pentafluorothio)-trifluoromethane, trifluoromethanesulfonylfluoride, bromodifluoronitrosomethane, bromofluoromethane, bromochlorofluoromethane, bromotrifluoromethane, chlorodifluoronitromethane, chlorodinitromethane, chlorofluoromethane, chlorotrifluoromethane, chlorodifluoromethane, dibromodifluoromethane, dichlorodifluoromethane, dichlorofluoromethane, difluoromethane, difluoroiodomethane, disilanomethane, fluoromethane, perfluoromethane, iodomethane, iodotrifluoromethane, nitrotrifluoromethane, nitrosotrifluoromethane, tetrafluoromethane, trichlorofluoromethane, trifluoromethane, 2-methylbutane, methyl ether, methyl isopropyl ether, methyllactate, methylnitrite, methylsulfide, methyl vinyl ether, neon, neopentane, nitrogen, nitrous oxide, 1,2,3-nonadecanetricarboxylic acid 2-hydroxytrimethyl ester, 1-nonene-3-yne, oxygen, 1,4-pentadiene, n-pentane, perfluoropentane, 4-amino-4-methylpentan-2-one, 1-pentene, 2-pentene (cis and trans), 3-bromopent-1-ene, perfluoropent-1-ene, tetrachlorophthalic acid, 2,3,6-trimethylpiperidine, propane, 1,1,1,2,2,3-hexafluoropropane, 1,2-epoxypropane, 2,2-difluoropropane, 2-aminopropane, 2-chloropropane, heptafluoro-1-nitropropane, heptafluoro-1-nitrosopropane, perfluoropropane, propene, hexafluoropropane, 1,1,1,2,3,3-hexafluoro-2,3-dichloropropane, 1-chloropropane, 1-chloropropylene, chloropropylene-(trans), chloropropane-(trans), 2-chloropropane, 2-chloropropylene, 3-fluoropropane, 3-fluoropropylene, perfluoropropylene, perfluorotetrahydropyran, perfluoromethyltetrahydrofuran, perfluorobutylmethylether, perfluoromethylpentylether, propyne, 3,3,3-trifluoropropyne, 3-fluorostyrene, sulfur (di)-decafluoride (S₂F₁₀), sulfur hexafluoride, 2,4-diaminotoluene, trifluoroacetonitrile, trifluoromethyl peroxide, trifluoromethyl sulfide, tungsten hexafluoride, vinyl acetylene, vinyl ether, xenon, 1-bromononafluorobutane, and perfluoroethers.

Preferred gases and gaseous precursors are compounds which are sparingly soluble in water but which may, in some cases, be lipid soluble, such as low molecular weight alkanes and their fluorinated analogs. Preferred gases and gaseous precursors include, but are not limited to, nitrogen, perfluorocarbons, sulfur hexafluoride, perfluoroether compounds and combinations thereof. The perfluorocarbons and perfluoroethers preferably have from 1 to 4 carbon atoms and from 4 to 10 fluorine atoms, most preferably perfluorobutane (C₄F₁₀). Preferred gaseous precursors generally have from about 4 to 8 carbon atoms, more preferably 5 or 6 carbon atoms, and from about 12 to 15 fluorine atoms. Perfluoroethers generally contain one or two oxygen atoms, preferably one oxygen atom. Preferred gaseous precursors include perfluoropentane, perfluorohexane, perfluorodecalin, perfluorotripropylamine, perfluorooctylbromide, perfluorobutylmethylether, perfluorotetrahydropyran, perfluoromethyltetrahydrofuran, perfluoromethylpentylether and other perfluoroether analogues containing between 4 and 6 carbon atoms, and optionally containing one halide ion, preferably Br¹⁻. Other examples of useful gaseous precursors include perfluoropropyloxylbromide and 2-bromooxyperfluoropropane. Also useful as gaseous precursors in the present invention are partially or fully fluorinated ethers, preferably having a boiling point of from about 36°C. to about 60 °C. Fluorinated ethers are ethers in which one or more hydrogen atoms is replaced by a fluorine atom.

The gas may comprise a fluorinated gas, which includes gases containing one or more than one fluorine atom. Preferred are gases which contain more than one fluorine atom, with perfluorocarbons (fully fluorinated fluorocarbons) being more preferred. The perfluorocarbon gas may be saturated, unsaturated or cyclic, including, but not limited to, perfluoromethane, perfluoroethane, perfluoropropane, perfluorocyclopropane, perfluorobutane, perfluorocyclobutane, perfluoropentane, perfluorocylcopentane, perfluorohexane, perfluoroheptane, perfluorooctane, perfluorononane, and mixtures thereof. Mixtures of different types of gases, such as mixtures of a perfluorocarbon gas and another type of gas, such as, for example, air or nitrogen, can also be used in the compositions. Other gases, including the gases exemplified above, would be apparent to one skilled in the art in view of the present disclosure.

The gases and/or gaseous precursors are incorporated in the targeted therapeutic delivery systems irrespective of the physical nature of the composition. Thus, it is contemplated that the gases and/or gaseous precursors may be incorporated, for example, in a surfactant randomly, such as emulsions, dispersions or suspensions, as well as in carriers, including vesicles which are formulated from lipids, such as micelles, liposomes, dendrimer, and polymer shells. Incorporation of the gases and/or gaseous precursors in the surfactant may be achieved by using any of a number of methods. For example, in the case of vesicles based on lipids, the formation of gas filled vesicles can be achieved by shaking or otherwise agitating an aqueous mixture which comprises a gas and/or gaseous precursor and one or more lipids. This promotes the formation of stabilized vesicles within which the gas and/or gaseous precursor is encapsulated.

Embodiments include the gases and/or gaseous precursors incorporated in vesicle compositions, with micelles and liposomes being preferred. Vesicles in which a gas or gaseous precursor or both are encapsulated are advantageous in that they provide improved reflectivity in vivo.

### V. Targeted Release of Drug Molecules

In one embodiment of the present invention, methods and apparatus are provided to achieve targeted release and activation of drug molecules at a specific site inside of a host. The drugs may be incorporated in a deactivated form and then transported to the site of the diseased tissue by the blood stream. Targeting of pressure pulses is done by the means of a focused pressure pulse or ultrasound source capable of the generation of acoustic waves at a peak tensile pressure of -5 MPa to -15 MPa to induce cavitation. Only at the focus of this source is the sound energy high enough for cavitational activity. Thus only at this site, the bonds of the target molecules are broken, thereby activating the drug molecules, which can then penetrate the target cells due to the membrane permeabilization mechanism of the cavitation.

The drugs or agents delivered into the host are preferably encapsulated or trapped within a structure thus rendering them inactive. Alternatively, the drug or agent is bound to another molecule or molecules such that the active portion of the drug or agent is unable to bind to receptors within the host, thus rendering it inactive. The drug or agent trapped within confining molecular structures is subsequently released by the application of the acoustic energy and the subsequent initiation ofthe cavitation process.

In one preferred embodiment, a method for initiating or deactivating chemical reactions inside a host comprising applying acoustic waves to the host of a sufficient amplitude to induce cavitation. The cavitation process produces cavitation bubbles that emit ultra short light pulses upon collapse within the infrared, visible and ultraviolet range which in turn activate or deactivate chemical reactions that are sensitive to ultra short light pulses within the infrared, visible or ultraviolet ranges. In one aspect of the invention, the chemical reaction comprises the three-dimensional folding or assembly of a drug or biologic agent. In another aspect of the invention, the drug or biologic agent combines with one or more ions or free radicals generated during the collapse of the cavitation bubbles. The chemical reaction also includes the breaking of a bond which would then release a prodrug, activated drug or other biological agent to a desired region within the host.

The target is optionally penetrated by the molecules due to transient permeabilization of the cell membranes by the cavitation effects. Alternatively, the drug or agent is delivered extracellularly or outside of the desired target or targets. For example, the drug is delivered in the blood stream or injected around the site of a tumor and then released where it acts upon the tumor without actually entering the tumor cells.

Alternatively, it may be desirable to deliver the drug or agent intracellularly.

Another application of the present invention is the controlled release of hormones and related bioactive substances for a variety of bodily control functions. A nearly constant rate of release can be obtained through proper design of an encapsulated hormone inside an encapsulating material. Alternately, a molecular structure can be synthesized so that fragmentation produces the desired hormones.

In either case a constant rate of release of hormones and related bioactive substances can be obtained. Such structures thus offer great promise for application to time-release drug formulations and to transdermal patches for application of drug materials. As conventional approaches to these applications yield a dosage rate which falls off with time, use of the present invention would improve the constancy of the dosage rate.

### VI. Therapeutic Agents

The methods and apparatus of the current invention contemplate the use of a virtually unlimited number of biological and pharmaceutical agents. These can include but are not limited to pharmaceuticals, blood products, genetic material, nutriceuticals, ions, radiopharmaceticals, immunostimulatory agents, antibodies or fragments thereof, and/or receptor proteins. These will be described in more detail below. One skilled in the are will quickly recognize that the lists of such agents given below are by no means exhaustive and are provided solely for illustrative purposes.

Such suitable pharmaceutical agents include, antifungal agents, antineoplastic agents, such as platinum compounds (e.g., spiroplatin, cisplatin, and carboplatin), methotrexate, adriamycin, taxol, mitomycin, ansamitocin, bleomycin, cytosine arabinoside, arabinosyl adenine, mercaptopolylysine, vincristine, busulfan, chlorambucil, melphalan, phenylalanine mustard (PAM)), mercaptopurine, mitotane, procarbazine hydrochloride, dactinomycin (actinomycin D), daunorubicin hydrochloride, doxorubicin hydrochloride, mitomycin, plicamycin, aminoglutethimide, estramustine phosphate sodium, flutamide, leuprolide acetate, megestrol acetate, tamoxifen citrate, testolactone, trilostane, amsacrine, asparaginase (L-asparaginase) Erwina asparaginase, etoposide (VP-16), interferon .alpha.-2a, interferon .alpha.-2b, teniposide (VM-26), vinblastine sulfate (VLB), vincristine sulfate, bleomycin, bleomycin sulfate, methotrexate, adriamycin, carzelesin, and arabinosyl; muramyldipeptide, muramyltripeptide, prostaglandins, microbial cell wall components, lymphokines, sub-units of bacteria, the synthetic dipeptide N-acetyl-muramyl-L-alanyl-D-isoglutamine; anti-fungal agents such as ketoconazole, nystatin, griseofulvin, flucytosine (5-fc), miconazole, amphotericin B, ricin, and beta-lactam antibiotics. Other agents include anti-allergic agents, anti-coagulation agents such as phenprocoumon and heparin; circulatory drugs such as propranolol; metabolic potentiators such as glutathione; antituberculars such as para-aminosalicylic acid, isoniazid, capreomycin sulfate cycloserine, ethambutol hydrochloride ethionanide, pyrazinamide, rifampin, and streptomycin sulfate; antivirals such as acyclovir, amantadine azidothymidine (AZT or Zidovudine), ribavirin, amantadine, vidarabine, and vidarabine monohydrate (adenine arabinoside, ara-A); antianginals such as diltiazem, nifedipine, verapamil, erythrityl tetranitrate, isosorbide dinitrate, nitroglycerin (glyceryl trinitrate) and pentaerythritol tetranitrate; anticoagulants such as phenprocoumon, heparin; antibiotics such as dapsone, chloramphenicol, neomycin, cefaclor, cefadroxil, cephalexin, cephradine erythromycin, clindamycin, lincomycin, amoxicillin, ampicillin, bacampicillin, carbenicillin, dicloxacillin, cyclacillin, picloxacillin, hetacillin, methicillin, nafcillin, oxacillin, penicillin G, penicillin V, ticarcillin, rifampin and tetracycline; antiinflammatories such as difimisal, ibuprofen, indomethacin, meclofenamate, mefenamic acid, naproxen, oxyphenbutazone, phenylbutazone, piroxicam, sulindac, tolmetin, aspirin and salicylates; antiprotozoans such as chloroquine, hydroxychloroquine, metronidazole, quinine and meglumine antimonate; antirheumatics such as penicillamine; narcotics such as paregoric and opiates such as codeine, heroin, methadone, morphine and opium; cardiac glycosides such as deslanoside, digitoxin, digoxin, digitalin and digitalis; neuromuscular blockers such as atracurium besylate, gallamine triethiodide, hexafluorenium bromide, metocurine iodide, pancuronium bromide, succinylcholine chloride (suxamethonium chloride), tubocurarine chloride and vecuronium bromide; sedatives/hypnotics such as amobarbital, amobarbital sodium, aprobarbital, butabarbital sodium, chloral hydrate, ethchlorvynol, ethinamate, flurazepam hydrochloride, glutethimide, methotrimeprazine hydrochloride, methyprylon, midazolam hydrochloride, paraldehyde, pentobarbital, pentobarbital sodium, phenobarbital sodium, secobarbital sodium, talbutal, temazepam and triazolam; local anesthetics such as bupivacaine hydrochloride, chloroprocaine hydrochloride, etidocaine hydrochloride, lidocaine hydrochloride, mepivacaine hydrochloride, procaine hydrochloride and tetracaine hydrochloride; general anesthetics such as droperidol, etomidate, fentanyl citrate with droperidol, ketamine hydrochloride, methohexital sodium and thiopental sodium.

Pharmaceutical agents also include hormones and hormone analogues and derivatives such as growth hormone, melanocyte stimulating hormone, estradiol, beclomethasone dipropionate, betamethasone, betamethasone acetate and betamethasone sodium phosphate, vetamethasone disodium phosphate, vetamethasone sodium phosphate, cortisone acetate, dexamethasone, dexamethasone acetate, dexamethasone sodium phosphate, flunsolide, hydrocortisone, hydrocortisone acetate, hydrocortisone cypionate, hydrocortisone sodium phosphate, hydrocortisone sodium succinate, methylprednisolone, methylprednisolone acetate, methylprednisolone sodium succinate, paramethasone acetate, prednisolone, prednisolone acetate, prednisolone sodium phosphate, prednisolone tebutate, prednisone, triamcinolone, triamcinolone acetonide, triamcinolone diacetate, triamcinolone hexacetonide, fludrocortisone. acetate, progesterone, testosterone, and adrenocorticotropic hormone.

The methods of the current invention also contemplate the delivery of vitamins such as cyanocobalamin neinoic acid, retinoids and derivatives such as retinol palmitate, .alpha.-tocopherol, naphthoquinone, cholecalciferol, folic acid, and tetrahydrofolate

Biological agents include peptides, polypeptides, proteins and the like including but not limited to angiostatin, manganese super oxide dismutase, tissue plasminogen activator, glutathione, insulin, peptides with affinity for the GPIIbIIIa receptor, opioid peptides, human chorionic gonadotropin, corticotropin release factor, cholecystokinins, bradykinins, bradykinin promoters, bradykinin inhibitors, elastins, vasopressin, pepsins, glucagon, substance P, neurokinin B, senktide, somatostatin, integrins, angiotensin converting enzyme inhibitors, oxytocin, calcitonins, IgG, IgA, IgM, thrombin, streptokinase, urokinase, protein kinase C, interferons, granulocyte colony stimulating factors, macrophage colony stimulating factors, tumor necrosis factors, nerve growth factors, platelet derived growth factors, lymphotoxin, epidermal growth factors, fibroblast growth factors, vascular endothelial cell growth factors, erythropoeitin, transforming growth factors, oncostatin M, interleukin 1, interleukin 2, interleukin 3, interleukin 4, interleukin 5, interleukin 6, interleukin 7, interleukin 8, interleukin 9, interleukin 10, interleukin 11, and interleukin 12, metalloprotein kinase ligands, and collagenases. Proteins also include enzymes such as alkaline phosphatase and cyclooxygenases.

Radiopharmaceuticals include radioactive particles or ions such as strontium, iodide rhenium, technetium, cobalt, yttrium or any pharmaceutical derivative thereof.

Other preferred therapeutics include genetic material such as nucleic acids, RNA, and DNA, of either natural or synthetic origin, including recombinant RNA and DNA. Types of genetic material that may be used include, for example, genes carried on expression vectors such as plasmids, phagemids, cosmids, yeast artificial chromosomes (YACs), and defective or "helper" viruses, both single and double stranded RNA and DNA and analogs thereof, such as phosphorothioate and phosphorodithioate oligodeoxynucleotides. Additionally, the genetic material may be combined, for example, with proteins or other polymers. Other examples of genetic material include antisense strands of DNA or RNA, cDNA, and mRNA

Fluorescent and radioactive dyes are also useful in the present invention. For example, one may wish to identify diseased tissue, tumors or the like in a particular region of a host's body. Dyes are incorporated within the encapsulation methods described herein and can be released at their desired location within the host. Dyes useful in the present invention include sudan black, fluorescein, R-Phycoerythrin, texas red, BODIPY FL, oregon green, rhodamine red-X, tetramethylrhodamine, BODIPY TMR, BODIPY-TR, YOYO-1, DAPI, Indo-1, Cascade blue, fura-2, amino methylcoumarin, FM1-43, NBD, carbosy-SNARF, lucifer yellow, dansyl+R--NH.sub.2, propidium iodide, methylene blue, bromocresol blue, acridine orange, bromophenol blue, 7-amino-actinomycin D, allophycocyanin, 9-azidoacridine, benzoxanthene-yellow, bisbenzidide H 33258 fluorochrome, 3HCl, 5-carboxyfluorescein diacetate, 4-chloro-1-naphthol, chromomycin-A₃, DTAF, DTNB, ethidium bromide, fluorescein-5-maleimide diacetate, mithramycin A, rhodamine 123, SBFI, SIST, tetramethylbenzidine, tetramethyl purpurate, thiazolyl blue, TRITC, and the like. Fluorescein may be fluorescein isothiocyanate. The fluorescein isothiocyanate, includes, inter alia, fluorescein isothiocyanate albumin, fluorescein isothiocyanate antibody conjugates, fluorescein isothiocyanate .alpha.-bungarotoxin, fluorescein isothiocyanate- casein, fluorescein isothiocyanate-dextrans, fluorescein isothiocyanate―insulin, fluorescein isothiocyanate--Lectins, fluorescein isothiocyanate--peroxidase, and fluorescein isothiocyanate--protein A.

Other drugs or agents useful in the current invention also include any natural or artificial blood product including, but not limited to, parenteral iron, hemin, hematoporphyrins and their derivatives, hemoglobin and myoglobin.

Still other drugs or agents useful in the current invention include any type of nutritional supplement, i.e. nutriceutical, including but not limited to, vitamins, minerals, herbal extracts, plant or animal extract. Other nutritional products can include parenteral nutritional agents such as Intralipid-R, Nutralipid-R, Liposyn III, and the like may be used as the carrier of the drug particles. Alternatively, if the biocompatible liquid contains a drug-solubilizing material such as soybean oil (e.g., as in the case of Intralipid), the drug may be partially or completely solubilized within the carrier liquid, aiding its delivery.

Any of the drugs or biological agents employed in the invention are administered to the host by a variety of different means depending upon the intended application. As one skilled in the art would recognize, administration can be carried out in various fashions, for example, topically, including ophthalmic, dermal, ocular and rectal, intrarectally, transdermally, orally, intraperitoneally, parenterally, intravenously, intralymphatically, intratumorly, intramuscularly, interstitially, intraarterially, subcutaneously, intraocularly, intrasynovially, transepithelially, pulmonarily via inhalation, ophthalmically, sublingually, buccally, or via nasal inhalation via insufflation, nebulization, such as by delivery of an aerosol. In the case of inhalation, a gaseous precursor delivered with a composition of the present invention such that the gaseous precursor is in liquid, gas, or liquid and gas form.

Any of the drugs used in the current invention may be administered as a prodrug that can be released and activated within the host. As a non-limiting example, prodrugs formulated with penetration enhancing agents, known to those skilled in the art and described above, may be administered transdermally in a patch or reservoir with a permeable membrane applied to the skin. The use of rupturing ultrasound may increase transdermal delivery of such therapeutic compounds. Further, an imaging mechanism may be used to monitor and modulate delivery of the prodrugs. For example, diagnostic ultrasound may be used to visually monitor the bursting of the gas filled vesicles and modulate drug delivery and/or a hydrophone may be used to detect the sound of the bursting of the gas filled vesicles and modulate drug delivery.

The useful dosage to be administered and the particular mode of administration will vary depending upon the age, weight and the particular mammal and region thereof to be scanned, and the particular contrast agent employed. Typically, dosage is initiated at lower levels and increased until the desired contrast enhancement is achieved. Various combinations of the lipid compositions may be used to alter properties as desired, including viscosity, osmolarity or palatability.

The size of the stabilizing materials and/or vesicles of the present invention will depend upon the intended use. With smaller liposomes, resonant frequency ultrasound will generally be higher than for the larger liposomes. Sizing also serves to modulate resultant liposomal biodistribution and clearance. In addition to filtration, the size of the liposomes can be adjusted, if desired, by procedures known to one skilled in the art, such as shaking, microemulsification, vortexing, filtration, repeated freezing and thawing cycles, extrusion, extrusion under pressure through pores of a defined size, sonication, homogenization, the use of a laminar stream of a core of liquid introduced into an immiscible sheath of liquid. See, for example, U.S. Pat. Nos. 4,728,578, 4,728,575, 4,737,323, 4,533,254, 4,162,282, 4,310,505 and 4,921,706; U.K. Patent Application GB 2193095 A; International Applications PCT/US85/01161 and PCT/US89/05040; Mayer et al., Biochimica et Biophysica Acta, 858:161-168 (1986); Hope et al., Biochimica et Biophysica Acta, 812:55-65 (1985); Mayhew et al., Methods in Enzymology, 149:64-77 (1987); Mayhew et al., Biochimica et Biophysica Acta, 755:169-74 (1984); Cheng et al, Investigative Radiology, 22:47-55 (1987); and Liposomes Technology, Gregoriadis, G., ed., Vol. 1, pp. 29-37, 51-67 and 79-108 (CRC Press Inc, Boca Raton, Fla., 1984).

Since vesicle size influences biodistribution, different size vesicles may be selected for various purposes. For example, for intravascular application, the preferred size range is a mean outside diameter between about 30 nm and about 10 µm, with the preferable mean outside diameter being about 5 µm. More specifically, for intravascular application, the size of the vesicles is preferably about 10 µm or less in mean outside diameter, and preferably less than about 7 µm, and more preferably less than about 5 µm in mean outside diameter. Preferably, the vesicles are no smaller than about 30 nm in mean outside diameter. To provide therapeutic delivery to organs such as the liver and to allow differentiation of tumor from normal tissue, smaller vesicles, between about 30 nm and about 100 nm in mean outside diameter, are preferred. For embolization of a tissue such as the kidney or the lung, the vesicles are preferably less than about 200 µm in mean outside diameter. For intranasal, intrarectal or topical administration, the vesicles are preferably less than about 100 µm in mean outside diameter. Large vesicles, between 1 and about 10 µm in size, will generally be confined to the intravascular space until they are cleared by phagocytic elements lining the vessels, such as the macrophages and Kupffer cells lining capillary sinusoids. For passage to the cells beyond the sinusoids, smaller vesicles, for example, less than about 1 µm in mean outside diameter, e.g., less than about 300 nm in size, may be utilized. In preferred embodiments, the vesicles are administered individually, rather than embedded in a matrix, for example.

For in vitro use, such as cell culture applications, the gas filled vesicles may be added to the cells in cultures and then incubated. Subsequently sonic energy can be applied to the culture media containing the cells and liposomes.

In carrying out the imaging methods of the present invention, the stabilizing materials and vesicle compositions can be used alone, or in combination with diagnostic agents, bioactive agents or other agents. Such other agents include excipients such as flavoring or coloring materials.

As described in greater detail herein, the particular therapeutic agent or drug to be delivered may be embedded within the wall of the vesicle, encapsulated in the vesicle and/or attached to the surface of the vesicle. The phrase "attached to" or variations thereof, as used herein in connection with the location of the agent, means that the agent is linked in some manner to the inside and/or the outside wall of the microsphere, such as through a covalent or ionic bond or other means of chemical or electrochemical linkage or interaction. The phrase "encapsulated in variations thereof' as used in connection with the location of the bioactive agent denotes that the bioactive agent is located in the internal microsphere void. The phrase "embedded within" or variations thereof as used in connection with the location of the bioactive agent, signifies the positioning of the bioactive agent within the vesicle wall(s) or layer(s). The phrase "comprising a bioactive agent" denotes all of the varying types of positioning in connection with the vesicle. Thus, the bioactive agent can be positioned variably, such as, for example, entrapped within the internal void of the gas filled vesicle, situated between the gas and the internal wall of the gas filled vesicle, incorporated onto the external surface of the gas filled vesicle, enmeshed within the vesicle structure itself and/or any combination thereof. The delivery vehicles may also be designed so that there is a symmetric or an asymmetric distribution of the drug both inside and outside of the stabilizing material and/or vesicle.

### VII. Targeted Drug Delivery or Chemical Activation Outside of the Host

An alternative embodiment of the current invention includes the activation or deactivation of a chemical reaction or the delivery of a drug or biological agent to a host while the host's blood is directed to an external apparatus. Thus, the host's blood is directed outside the host to a fluid chamber at the focus of the acoustic source where the inactivated drug or agent is mixed with the host's blood in the fluid chamber. The mixture is then guided through the focus of the acoustic source, where it receives the acoustic pulses in order to activate the drug or agent. The host's blood is then redirected to the host. In order to enhance the cavitation process within the fluid chamber, cavitation-enhancing seeds are optionally added to the fluid chamber.

### VIII. The Acoustic Source Within the Host

In yet another, a drug or biological agent can be formulated and delivered as described by any means herein and an acoustic source can be placed for example via venous or arterial catheterization directly into a blood vessel at or near the desired target for the drug or biological agent. Thus, the acoustic source is placed directly into a blood vessel, the inactivated drug molecule is supplied to the blood stream and then the drug is activated at the cavitational zone at the tip of the focus of the acoustical source. For example, one such condition that is amenable to a procedure of the above type is circulatory thrombosis and embolism. Thrombosis is a condition where there are clots in the circulatory system attached to the walls of the blood vessels; such a clot becomes an embolism when it breaks free to become lodged somewhere else in the circulatory system. Typically, emergency conditions involving obstruction of circulation due to thrombosis or embolism are usually treated surgically (perhaps in combination with clot-dissolving drugs, such as heparin), whereas the treatment of non-emergency cases usually focuses on systemic injection of anticoagulant and/or clot-dissolving drugs. The present invention can be used to greatly reduce the risk of side and or toxic effects of the drugs used. A systemic dose of encapsulated anticoagulant or clot-dissolving drugs is injected and has no systemic effect on the body. A catheter equipped with an acoustic energy source is threaded to the site of the clot, and oriented so that the focus in on the region of the clot. The cavitation induced by the acoustic energy releases the active drugs in the immediate vicinity of the clot, bathing the clot in a much higher dose than can safely be applied to the entire circulatory system. The final state, once the clot has been dissolved, is that there is a small systemic dose of anticoagulant which continues to act on the clot, but which is much less dangerous than a systemic dose which would have produced equivalent doses to the clot in the early stages of treatment. Alternatively, if a blood vessel is completely blocked, this treatment will be much less effective, as blood flow will not efficiently bring fresh confined drug into the vicinity of the clot to be released. This can be countered by injecting the encapsulated drug molecules at the site of the clot, e.g., through a catheter.

Another preferred embodiment involves an implantable drug delivery device or "biochip" comprising a miniaturized electronic circuit chip, a sensor to detect when the host is in need of the particular drug or biological agent, a drug or agent storage means and a means for the generation of the acoustic waves inside a channel, wherein the channel guides a fluid containing the drug or agent into the host's blood stream or tissue. The acoustic wave generator is capable of generating acoustic waves at a peak tensile pressure of -5 MPa to -15 MPa to induce cavitation. Thus, as a non-limiting example, the drug delivery device can be implanted within a host that suffers from diabetes. The sensor can be equipped to detect low levels of blood glucose (or insulin) and at a certain level will activate the storage means to release the drug, further activating an acoustic energy source, generating a cavitational zone to release the drug or biological agent into the host.

### IX. Imaging

Any of the methods described herein, can be utilized to image a patient generally or to diagnose the presence of diseased tissue in a patient. The imaging process of the present invention may be carried out by administering an encapsulated imaging agent to a patient that is released at a desired location or locations and then scanning the patient using, for example, ultrasound, computed tomography, and/or magnetic resonance imaging, to obtain visible images of an intemal region of a patient and/or of any diseased tissue in that region.

The present invention also provides a method of diagnosing the presence of diseased tissue via the delivery and imaging of various targeting ligands or agents including but not limited to monoclonal antibodies, radiopharmaceuticals, or receptors to which a florescent or radioimaging agent is attached

Nuclear Medicine Imaging (NMI) may-also be used in connection with the diagnostic and therapeutic method aspects of the present invention. For example, NMI may be used to detect radioactive gases, such as Xe¹³³, which may be incorporated in the present compositions in addition to, or instead of, the gases discussed above. Such radioactive gases may be entrapped within vesicles for use in detecting, for example, thrombosis. Preferably, bifunctional chelate derivatives are incorporated in the walls of vesicles, and the resulting vesicles may be employed in both NMI and ultrasound. In this case, high energy, high quality nuclear medicine imaging isotopes, such as technetium⁹⁹ or indium¹¹¹ can be incorporated in the walls of vesicles. Whole body gamma scanning cameras can then be employed to rapidly localize regions of vesicle uptake in vivo. If desired, ultrasound may also be used to confirm the presence, for example, of a clot within the blood vessels, since ultrasound generally provides improved resolution as compared to nuclear medicine techniques. NMI may also be used to screen the entire body of the patient to detect areas of vascular thrombosis, and ultrasound can be applied to these areas locally to promote rupture of the vesicles and treat the clot.

For optical imaging, optically active gases, such as argon or neon, may be incorporated in any of the encapsulating materials described herein. In addition, optically active materials, for example, fluorescent materials, including porphyrin derivatives, may also be used. Elastography is an imaging technique which generally employs much lower frequency sound, for example, about 60 KHz, as compared to ultrasound which can involve frequencies of over 1 MHz. In elastography, the sound energy is generally applied to the tissue and the elasticity of the tissue may then be determined. In connection with preferred embodiments of the invention, which involve highly elastic vesicles, the deposition of such vesicles onto, for example, a clot, increases the local elasticity of the tissue and/or the space surrounding the clot. This increased elasticity may then be detected with elastography. If desired, elastography can be used in conjunction with other imaging techniques, such as MRI and ultrasound.

### X. Use of Stabilizing Materials

Stabilizing materials may also be employed, if desired, in connection with computed tomography (CT) imaging, magnetic resonance imaging (MRI), optical imaging, or other of the various forms of diagnostic imaging that are well known to those skilled in the art. For optical imaging, gas bubbles improve visualization of, for example, blood vessels on the imaging data set. With CT, for example, if a high enough concentration of the present contrast media, and especially gas filled vesicles, is delivered to the region of interest, for example, a blood clot, the clot can be detected on the CT images by virtue of a decrease in the overall density of the clot. In general, a concentration of about 1/10 of 1% of gas filled vesicles or higher (on a volume basis), may be needed to delivered to the region of interest, including the aforementioned blood clot, to be detected by CT.

Examples of suitable contrast agents for use in combination with the present stabilizing materials include but are not limited to, stable free radicals, such as, stable nitroxides, as well as compounds comprising transition, lanthanide and actinide elements, which may, if desired, be in the form of a salt or may be covalently or non-covalently bound to complexing agents, including lipophilic derivatives thereof, or to proteinaceous macromolecules. Preferable transition, lanthanide and actinide elements include, for example, Gd(III), Mn(II), Cu(II), Cr(III), Fe(II), Fe(III), Co(II), Er(II), Ni(II), Eu(III) and Dy(III). More preferably, the elements may be Gd(III), Mn(II), Cu(II), Fe(II), Fe(III), Eu(III) and Dy(III), most preferably Mn(II) and Gd(III). The foregoing elements may be in the form of a salt, including inorganic salts, such as a manganese salt, for example, manganese chloride, manganese carbonate, manganese acetate, and organic salts, such as manganese gluconate and manganese hydroxylapatite, salts of iron, such as iron sulfides, and ferric salts, such as ferric chloride.

### XI. Gases and Gaseous Precursors

The present targeted therapeutic delivery systems can optionally include a gas, such as an inert gas. The gas provides the targeted therapeutic delivery systems with enhanced reflectivity, particularly in connection with targeted therapeutic delivery systems in which the gas is entrapped within the carrier. This may increase their effectiveness as contrast agents or delivery vehicles.

Preferred gases are inert and biocompatible, and include, for example, air, noble gases, such as helium, rubidium, hyperpolarized xenon, hyperpolarized argon, hyperpolarized helium, neon, argon, xenon, carbon dioxide, nitrogen, fluorine, oxygen, sulfur-based gases, such as sulfur hexafluoride and sulfur tetrafluoride, fluorinated gases, including, for example, partially fluorinated gases or completely fluorinated gases, and mixtures thereof. Fluorinated gases include fluorocarbon gases, such as perfluorocarbon gases and mixtures thereof.

In certain preferred embodiments, a gas, for example, air or a perfluorocarbon gas, is combined with a liquid perfluorocarbon, such as perfluoropentane, perfluorohexane, perfluoroheptane, perfluorodecalin, perfluorododecalin, perfluorooctyliodide, perfluorooctylbromide, perfluorotripropylamine and perfluorotributylamine.

This invention may, however, be embodied in many different forms and should not be construed as limited to the embodiments set forth herein; rather, these embodiments are provided so that this disclosure is thorough and complete, and fully conveys the scope of the invention to those skilled in the art. Modifications and other embodiments of the invention will become apparent to one skilled in the art to which this invention pertains having the benefit of the teachings presented in the foregoing descriptions and associated drawings. It is to be understood that the invention is not limited to the specific embodiments disclosed and that modifications and other embodiments are intended to be included within the scope of the appended claims.

Cavitation, which is the occurrence of bubbles in fluids, may be generated by rarefaction phases of sound signals. Pre-existing cavitation seeds are agitated by the sound waves. During rarefaction phases of the sound, they tart expansion. After reaching a quasistable size around the maximum radius of the bubbles, they collapse. When collapsing, the stored energy is released as a burst of energy comprising acoustic energy in the form of a shock wave and electromagnetic energy in the form of a light flash. Therefore, one aspect of the present invention is to generate the flashes of light for the breaking of chemical bonds by the collapse of cavitation bubbles.

Another aspect of the present invention is the property of cavitation of transient permeabilization of the membranes of cells. The membranes stay permeable only for a period of some milliseconds to seconds, which is long enough to allow for the entrance of molecules, which would otherwise be kept out. Examples of these molecules include drugs, genes, proteins, etc.

In the present invention, the properties of cavitation of transient permeabilization of cell membranes is combined with the ability to activate chemical reactions like the breaking of bonds by the light energy of the collapsing cavitation bubbles for medical and biological use.

In a medical application, this method can be used to achieve targeted release and activation of drug molecules at a specific site inside the body. The drugs may be incorporated in a deactivated form, e.g., intravenous. It is transported to the site of the diseased tissue by the blood stream. Targeting of pressure pulses is done by the means of a focused pressure pulse or ultrasound source. Only at the focus of this source is the sound energy high enough for cavitational activity. Thus only at this site, the bonds of the target molecules are broken, thereby activating the drug molecules, which can then penetrate the target cells due to the membrane permeabilization mechanism of the cavitation.

Cavitation seeds may be introduced in the form of gas-filled microspheres, e.g., liposomes. The spectral content of the light pulse may be determined by the mixture of gas inside the bubbles. In medical use, the patient could also breathe in an appropriate gas, which would then change the spectral content of collapsing cavitation bubble light. Cavitation seeds may also be generated by acoustic pulses or pulse sequences, as welt as continuous ultrasound of sufficient amplitude. We have found that it is not necessary to use shockwaves to achieve the desired cavitational action. It is sufficient to exceed the cavitation threshold in the fluid by applying ultrasound pulses including a rarefaction phase of ca. - 12 Mpa in water.

In another biologic application, the molecules and the cells are mixed in a fluid chamber, which also includes the focus of the acoustic source. This fluid chamber may be any closed (Vial) or open (Tube) volume. For example, patient blood can be guided through a tube inside the claimed apparatus, where it is mixed with molecules including the desired drug, which is inactivated. Additionally, cavitation-enhancing seeds, e.g., ultrasound contrast agents, can be supplied. The blood/molecule mixture is then guided through the focus of the acoustic source, where it receives the acoustic pulses. Here cavitation-induced breaking of the bonds is then activating the drug molecules, which penetrate the cells inside the blood stream. Another favorable method would include an appropriate acoustic source, which is generating sound energy sufficient to support cavitation inside a blood vessel. Upstream from the cavitation area, the inactivated molecule is supplied to the bloodstream, e.g., by a syringe. If the vessel leads to a tumor, and the cavitation zone is placed at the periphery or inside the tumor, the drug will be activated at the tumor cells, which are transiently permeable due to the cavitational effect.

Another preferred embodiment may be the miniaturization of the device as a "biochip". This chip contains bio-sensitive sensors, which are a combination of biomolecules and electronic circuits. The chip may also contain means for the generation of the acoustic waves inside a channel, which is guiding a fluid containing the molecules and/or cells to be analyzed. The acoustic waves may also be generated from a second generator, the cavitational zone preferably being dose to the biochip sensing area. It may contain a channel, which guides the fluid through the sensor area, where the cavitational zone is placed.

Medical applications include, but are not necessarily limited to, autoimmune diseases, genetic defects, cancerous tumors, treatment of blood, and suppression of immune reactions. Non-medical applications may be found in biotechnology for the selective treatment of plant and animal cells and diseases.

## Claims

1. Apparatus for targeted activation or deactivation of chemical reactions, comprising initiation means for initiating cavitation inside at least one medium, **characterized in that** said initiation means are adapted to generate photons by means of controlled cavitation for said activation or deactivation of chemical reactions within the at least one medium.

2. The apparatus according to claim 1, wherein said means for initiating cavitation are means for generating acoustic waves with compressive and/or tensile components having a specific peak compressive and tensile pressure.

3. The apparatus according to claim 1 or 2, wherein said means for initiating cavitation are non invasive means.

4. The apparatus according to claim 1 to 3, further comprising enhancing means for enhancing said controlled cavitation processes said enhancing means adapted for applying cavitation enhancing compounds or substances to said at least one medium.

5. The method according to claim 4, wherein said cavitation enhancing compounds or substances comprises gas filled microspheres, ultrasonic contrast agents, any inorganic gas filled molecule that does not reflect, any material with a low acoustic impedance and any material with a high acoustic impedance.

6. The apparatus according to any of claims 1 to 5, further comprising applying means for applying chemical substances to said at least one medium to be at least partly processed by said activated or deactivated chemical reactions.

7. The apparatus according to claim 6, wherein said chemical substances are delivered along with said cavitation enhancing compounds or substances.

8. The apparatus according to any of claims 1 to 7, further comprising manipulating means for manipulating a spectrum of said photons generated by said controlled cavitation processes.

9. The apparatus according to any of claims 1 to 8, wherein the initiation means initiate controlled cavitation inside at least one medium within a limited volume.

10. The apparatus according to any of claims 1 to 9, further comprising imaging means for displaying regions of interest of a living body usable for positioning said regions of interest of said living body for initiating cavitation within said regions of interest.

11. The apparatus according to any of claims 1 to 10, wherein the apparatus is used for medical therapy.

12. An implantable drug delivery device comprising:
i) a miniaturized electronic circuit chip
ii) a sensor,
iii) a drug or agent storage means and
iv) a means for the generation of the acoustic waves at a specific peak tensile pressure to induce cavitation.

13. The device according to claim 12 further comprising a second acoustic wave generator capable of generating a cavitational zone.

14. The device according to claim 13, wherein the second acoustic wave generator contains a fluid channel to guide the drug or agent through the cavitational zone.

15. An implantable bio-sensing device comprising:
i) a miniaturized electronic circuit chip
ii) a sensor
iii) a means for the generation of the acoustic waves at a specific peak tensile pressure to induce cavitation inside a channel, wherein the channel guides a fluid containing a substance or cells to be analyzed.

16. The device according to claim 15 further comprising a second acoustic wave generator capable of forming a cavitational zone close to the sensor.

17. The device according to claim 16, wherein the second acoustic wave generator contains a channel to guide a fluid or cells through the cavitational zone close to the sensor in order to detect properties of the fluid or cells which are agitated by the cavitational effects close to the sensor area.

18. Method for targeted activation or deactivation of chemical reactions, comprising initiating cavitation inside at least one medium, **characterized in** further comprising generating photons by means of controlled cavitation for said activation or deactivation of chemical reactions within the at least one medium.

19. The method according to claim 18, wherein said initiating cavitation is performed by applying acoustic waves with compressive and/or tensile components having a specific peak compressive and tensile pressure to said at least one medium.

20. The method according to claim 18 or 19, wherein said initiating cavitation is a non invasive initiating of the cavitation.

21. The method according to claim 18 to 20, further comprising enhancing said controlled cavitation processes for receiving a specific yield in cavitation by applying cavitation enhancing compounds or substances to said at least one medium.

22. The method according to claim 21, wherein said cavitation enhancing compounds or substances comprises gas filled microspheres, ultrasonic contrast agents, any inorganic gas filled molecule that does not reflect, any material with a low acoustic impedance and any material with a high acoustic impedance.

23. The method according to any of claims 18 to 22, further applying chemical substances to said at least one medium, wherein said chemical substances are at least partly processed by said activated or deactivated chemical reactions.

24. The method according to claim 23, wherein said chemical substances are delivered along with said cavitation enhancing compounds or substances.

25. The method according to any of claims 18 to 24, further comprising manipulating a spectrum of said photons generated by said controlled cavitation processes for receiving a specific yield in said chemical reactions.

26. The method according to any of claims 18 to 25, wherein said initiating cavitation inside at least one medium is limited to specific region.
